# EUROPEAN PATENT SPECIFICATION

(11) **EP 3 551 165 B1**
(45) Date of publication and mention of the grant of the patent: **01.07.2020**
(21) Application number: 18709375.2
(22) Date of filing: 14.03.2018
(51) Int. Cl.: A61K 31/715, A61K 36/48, A61K 38/16, A61K 47/55, A61K 47/61, A61K 47/64, A61P 17/00, A61P 17/06, A61K 9/00, A61K 9/06, A61K 9/107

(54) **TOPICAL PHARMACEUTICAL COMPOSITIONS AS WELL AS MEDICAL DEVICES COMPRISING THEREOF FOR THE TREATMENT OF SKIN DISORDERS**
TOPISCHE PHARMAZEUTISCHE ZUSAMMENSETZUNGEN SOWIE MEDIZINISCHE VORRICHTUNGEN DAVON ZUR BEHANDLUNG VON HAUTERKRANKUNGEN
COMPOSITIONS PHARMACEUTIQUES TOPIQUES AINSI QUE DES DISPOSITIFS MÉDICAUX LES COMPRENANT POUR LE TRAITEMENT DE TROUBLES CUTANÉS

(30) Priority: 14.03.2017 EP 17160717
(43) Date of publication of application: 16.10.2019
(73) Proprietor: DEVINTEC SAGL, 6900 Lugano (CH)
(72) Inventor: ALONSO COHEN, Miguel Angel, 08034 Barcelona (ES); DI FULVIO, Marco, 01038 Soriano Nel Cimino (IT)
(74) Representative: ZBM Patents - Zea, Barlocci & Markvardsen
(86) International application number: PCT/EP2018/056362
(87) International publication number: WO 2018/167131

(56) References cited:
- EP-A1- 3 103 438
- WO-A1-97/39757
- WO-A1-2017/207223
- TAMNAK SAHAR ET AL: "Physicochemical properties, rheological behavior and morphology of pectin-pea protein isolate mixtures and conjugates in aqueous system and oil in water emulsion", FOOD HYDROCOLLOIDS, ELSEVIER BV, NL, vol. 56, 4 January 2016 (2016-01-04), pages 405-416, XP029416151, ISSN: 0268-005X, DOI: 10.1016/J.FOODHYD.2015.12.033
- BAORU YIN ET AL: "Influence of Pea Protein Aggregates on the Structure and Stability of Pea Protein/Soybean Polysaccharide Complex Emulsions", MOLECULES, vol. 20, no. 3, 20 March 2015 (2015-03-20) , pages 5165-5183, XP055398788, DOI: 10.3390/molecules20035165
- JAE-HO YANG: "Topical Application of Fucoidan Improves Atopic Dermatitis Symptoms in NC/Nga Mice", PHYTOTHERAPY RESEARCH, vol. 26, no. 12, 19 March 2012 (2012-03-19), pages 1898-1903, XP055204203, ISSN: 0951-418X, DOI: 10.1002/ptr.4658

### SEQUENCE LISTING

<110> DEVINTEG SAGL
<120> Topical pharmaceutical or cosmetic compositions as well as medical devices comprising thereof for the treatment of skin disorders
<130> 3787PC00
<160> 1
<170> PatentIn version 3.5
<210> 1
   <211> 37
   <212> PRT
   <213> Artificial Sequence
<220>
   <223> LL-37 peptide
<400> 1
   Leu Leu Gly Asp Phe Phe Arg Lys Ser Lys Glu Lys Ile Gly Lys Glu
   1               5                   10                  15
   Phe Lys Arg Ile Val Gln Arg Ile Lys Asp Phe Leu Arg Asn Leu Val
               20                  25                  30
   Pro Arg Thr Glu Ser
           35

## Description

### Technical Field

The present invention relates topical compositions comprising a combination of pea protein and polysaccharide and to the application thereof in cosmetics and in the treatment of inflammatory skin disorders.

### Background Art

Inflammatory skin disorders refer to the majority of the most common rashes such as, for example, eczema, dermatitis and psoriasis. These diseases are usually chronic and mainly show the following symptoms: itching, redness, swelling, dryness and desquamation.

Atopic dermatitis (AD) is a type of inflammation of the skin (dermatitis). It results in itchy, red, swollen, and cracked skin. Clear fluid may come from the affected areas, which often thicken over time. The condition typically starts in childhood with changing severity over the years. In children under one year of age much of the body may be affected. As people get older, the back of the knees and front of the elbows are the most common areas affected. In adults the hands and feet are the most commonly affected areas. Scratching worsens symptoms and affected people have an increased risk of skin infections. Many people with atopic dermatitis develop hay fever or asthma.

Bacterial infections are common in atopic dermatitis. This is usually with staphylococcal or streptococcal bacteria (see staphylococcal skin infections and streptococcal skin infections). People who have atopic dermatitis are particularly prone to skin infections. This is in part due to the breaks in the skin from very dry, split skin and from scratching the itchy areas. People with atopic dermatitis also seem to have a reduced ability to fight against these common bacteria on the skin. As a result people with atopic dermatitis frequently suffer from boils, folliculitis and infections of their eczema. This begins a viscious cycle as infection causes the eczema to worsen and become more resistant to the usual treatment with emollients and topical steroids. Antibiotics are often required to eliminate the infection before the eczema can once more be brought under control.

AD treatment involves avoiding things that make the condition worse, daily bathing with application of a moisturising cream afterwards, applying steroid creams when flares occur, and medications to help with itchiness. Steroid pills or creams based on calcineurin inhibitors may occasionally be used if other measures are not effective. Antibiotics (either by mouth or topically) may be needed if a bacterial infection develops.

Psoriasis is a common skin disorder characterized by epidermal hyperplasia caused by aberrant hyperproliferation of the keratinocytes and by the presence of red scaly plaques. In this chronic disease, lesions are typically subject to remissions and exacerbations. There are several patterns; although plaque psoriasis is the most common, there is also psoriasis guttata, having lesions in the shape of water drops spread around the trunk and extremities, and pustular psoriasis, which are usually located on the palms of the hands and soles of the feet. The most common inflammatory lesions vary from discrete erythematous papules to plaques covered with silvery scales.

The symptoms of psoriasis need to be treated to control the severity and propagation frequency of the disease. There are different systemic and topical pharmacological treatments comprising retinoids, anti-inflammatory agents such as corticoids, and moisturizing agents of natural origin as aloe vera or rosehip oil.

However, in spite of the extensive scientific literature and numerous treatment strategies, there is still no effective treatment for psoriasis without side effects.

It has also been reported that patients suffering from cancer, when treated with chemo- or radiotherapy, can develop skin inflammation as side-effect.

The treatment of inflammatory skin disorders varies, depending on the cause and each person's experience of the condition. In addition to the lifestyle and home remedies, most treatment plans include one or more of the following: (a) applying corticosteroid creams, (b) applying certain creams or lotions that affect your immune system (calcineurin inhibitors), and (c) exposing the affected area to controlled amounts of natural or artificial light (phototherapy). Baoru Yin and colleagues (Baoru Y. *et al.,* 2015) disclose the topical administration of fucoidan in the treatment of atopic dermatitis.

Many alternative therapies, including those listed below, have helped some people manage their dermatitis (such as dietary supplements, rice bran broth, tea tree oil, fish oil supplements or aloe vera for seborrheic dermatitis) although no evidence for their effectiveness is conclusive. WO97/39757 discloses a tautening aqueous gel comprising a vegetable based tensor agent containing a protein-based complex of vegetable extract and polysaccharides, comprising pea protein and Sclerotium gum (VEGETENSOR.

Tamnak Sahar and colleagues (Tamnak Sahar et al., "Physicochemical properties, rheological behavior and morphology of pectin-pea protein isolate mictures and conjugates in aqueous system and oil in water emulsion", 2016, Food Hydrocolloids, 56, 405-416) discloses an O/W emulsion comprising pea protein and pectin. In section 2.2.2 it is disclosed how these emulsions are prepared, and it is pointed out that the protein and the pectin are mixed with potassium ascorbate and citric acid. It is well-established that potassium ascorbate gives rise to rashes. Consequently, the O/W emulsion disclosed in D2 cannot be considered as a topical composition to be used in therapy or cosmetics.

Baoru Yin and colleagues (Baoru Y. et al., "Influence of pea protein aggregates on the structure and stability of pea protein/soybean polysaccharide complex emulsions", 2015, Molecules, 20(3), 5165-5183) discloses emulsions of pea protein and a soja polysaccharide which is soluble at different pH, and provides data about how the pea protein stabilized the emulsion, something useful in food and beverage fields. D3 at least does not disclose a pharmaceutical composition comprising pea protein+polysaccharide which is useful in the treatment of skin barrier dysfunctions.

EP3103438 discloses the intranasal topical use of xyloglucan in the treatment of nasal congestion. In paragraph [0018] it is mentioned that xyloglucan can be combined with further ingredients, among which pea protein is mentioned. No example is provided supporting the combined use of polysaccharide+pea protein. JAE-HO YANG discloses (JAE-HO YANG et al, "Topical Application of Fucoidan Improves Atopic Dermatitis Symptoms in NC/Nga Mice",2012, PHYTOTHERAPY RESEARCH), (abstract): Fucoidan polysaccharide from seaweed in topical applications for the treatment of atopic dermatitis.

Therefore, there is still a need to find new efficient treatments for the treatment of inflammatory skin disorders.

### Summary of Invention

The present inventors have found that when a combination of pea protein and a polysaccharide is topically administered to an atopic dermatitis, psoriasis or rosacea animal model, there is a substantial restoration of the skin barrier function.

Main components of the skin barrier are found in the outer layers of the epidermis (such as filaggrin), the proteins that form the tight junction (TJ) and components of the innate immune system. TJ are formed by occludin, claudin, zonula occludens 1 (ZO1) and 2 (ZO2), junctional adhesion molecule-1 (JAM1) and the multi-PDZ-1 protein (MUPP1)

When an inflammatory process starts in the skin (such as an atopic dermatitis), the components forming the barrier are negatively affected, disturbing the appropriate layered-structre of the skin barrier, and giving raise to an uncontrolled permeability.

As provided below, it has been found that the topical administration of the combination of pea protein and a polysaccharide provides: a significant reduction in the alteration of zonula occludens and occluding localization, as well as the restoration of filaggrin and the substantial reduction in degranulation of mast cells.

Altogether these data are indicative that the topical administration of the combination substantially restores the skin barrier function due to a restoration of its main components (i.e., tight junction, filaggrins and components of innate immune system).

In addition to the above, it has been found that there is a reduction in the extension of skin damage as well as in erythemas.

Remarkably, the efficacy of the combination of pea protein and polysaccharide is of the same order as the one shown by hydrocortisone. This is of special relevance because it is well-known for those skilled in the art that hydrocortisone gives raise to many undesired side-effects, which limits its prescription by the physician.

Therefore, the present invention provides an alternative treatment to hydrocortisone, which is as effective as the latter and, at the same time, is more safety, not giving rise to the many side-effects reported with cortisone products.

The results provided herein allows concluding that the topical administration of a combination comprising pea protein and a polysaccharide can efficiently prevent and treat any skin disorder due to a skin barrier dysfunction.

Thus, in a first aspect the present invention provides a combination comprising pea protein and a polysaccharide for use in the prophylaxis and/or treatment of a skin disorder due to a skin barrier dysfunction. This aspect can alternatively be formulated as the use of a combination comprising pea protein and a polysaccharide for the manufacture of a medicament for the prophylaxis and/or treatment of a skin disorder due to a skin barrier dysfunction. This aspect can also be alternatively formulated as a method for the prophylaxis and/or treatment of a skin disorder due to a skin barrier dysfunction comprising the step of administering an effective amount of a combination comprising pea protein and a polysaccharide to a subject in need thereof.

As it is shown in Example 6 below, the present inventors found a remarkable improved effect when pea protein was topically administered in combination with the polysaccharide both in the histological score as well as in the expression of filaggrin. In addition, as it is shown below, the combination of the invention remarkably reduced the mast cell degranulation.

In a second aspect the present invention provides a topical pharmaceutical composition for use in the topical treatment or prevention of a skin disorder due to a skin barrier dysfunction, the composition comprising: (i) an effective amount of a combination of pea protein and polysaccharide; and (ii) one or more appropriate topical pharmaceutically or cosmetically acceptable excipients or carriers; or, alternatively, a topical pharmaceutical or cosmetic composition comprising: (i) an effective amount of a combination comprising pea protein and a polysaccharide which is selected from xyloglucan, fucoidan, ulvan and a mixture thereof; and (ii) one or more appropriate topical pharmaceutically or cosmetically acceptable excipients or carriers.

In a third aspect the present invention provides a topical medical device comprising the topical pharmaceutical or cosmetical composition according to the second aspect of the invention.

### Brief Description of Drawings

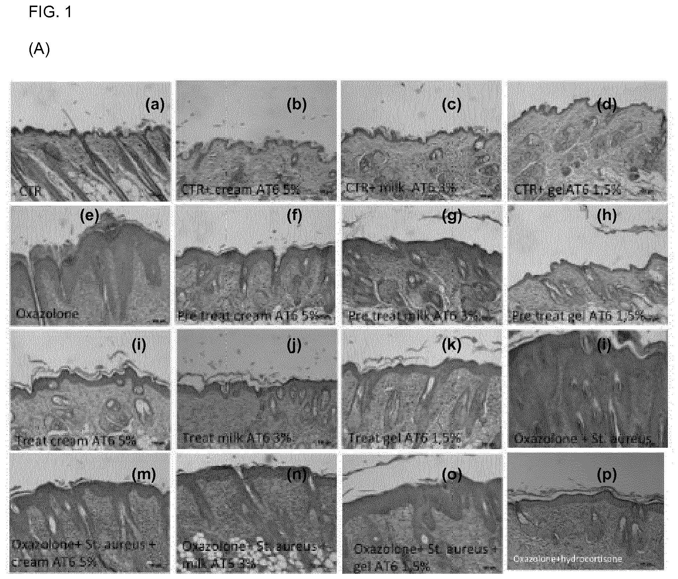

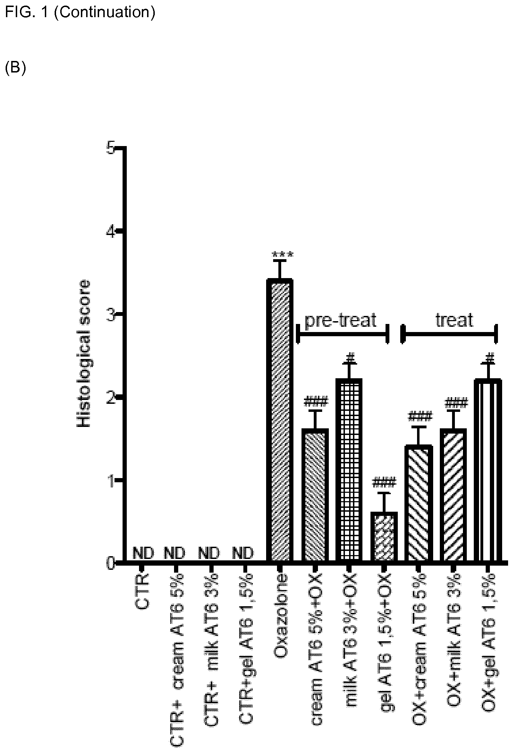

FIG. 1 : Histological avaluation
   (A) Immunostaining skin sections from mice (a) just receiving polysorbate; (b) just receiving a cream composition of the invention without oxazolone treatment for 4 weeks; (c) just receiving a lotion composition of the invention without oxazolone treatment for 4 weeks; (d) just receiving a gel composition of the invention without oxazolone treatment for 4 weeks; (e) receiving vehicle (polysorbate 80) with oxazolone treatment for 4 weeks; (f) receiving a cream composition of the invention 1 hr before oxazolone treatment for 4 weeks; (g) receiving a lotion composition of the invention 1 hr before oxazolone treatment for 4 weeks; (h) receiving a gel composition of the invention 1 hr before oxazolone treatment for 4 weeks; (i) receiving a cream composition of the invention after oxazolone treatment for 2 weeks; (j) receiving a lotion composition of the invention after oxazolone treatment for 2 weeks; (k) receiving a gel composition of the invention after oxazolone treatment for 2 weeks; (l) receiving subcutaneous inoculation of 50 µl of *Staphylococcus aureus* (St. aureus) in addition to oxazolone for 2 weeks; (m) receiving a cream composition of the invention 24 hrs after subcutaneous inoculation of *Staphylococcus aureus* bacterial suspension in addition to oxazolone for 2 weeks; (n) receiving a lotion composition of the invention 24 hrs after subcutaneous inoculation of *Staphylococcus aureus* bacterial suspension in addition to oxazolone for 2 weeks; (o) receiving a gel composition of the invention 24 hrs after subcutaneous inoculation of *Staphylococcus aureus* bacterial suspension in addition to oxazolone for 2 weeks; and (p) receive hydrocortisone (HC; 2.5 mg/mice) after oxazolone treatment for 4 weeks
   (B) Bar-representation of the histological score (Y-axis) vs. particular tested groups of mice (X-axis).
FIG. 2 Expression of ZO-1 in skin sections obtained from mice belonging to groups (a) to (p) as identified above in FIG. 1 (A).
FIG. 3 Expression of occludin in skin sections obtained from mice belonging to groups (a) to (p) as identified above in FIG. 1 (A).
FIG. 4. Expression of filaggrin in skin sections obtained from mice belonging to groups (a) to (p) as identified above in FIG. 1 (A).
FIG. 5. Represents the Erythema Index (EI) in Y-axis vs. particular tested groups of mice (X-axis).
FIG. 6: Mast cell quantification
   A. Immunostaining skin sections from mice belonging to groups (a) to (p) as defined above in FIG. 1(A).
   B. Bar-representation of the number of mast cells counted/mm² in skin sections obtained from particular tested groups of mice.
FIG. 7: Bar-representation of the BrdU labelling Index (Y-axis) for lymph nodes isolated from the groups of mice identifies in FIG. 1 (A) as groups (a) to (d) (X-axis).
FIG. 8 Immunostaining skin sections from mice's right flank: Group 1= mice received Vaseline cream without Imiquimod (IMQ) treatment for 5 days (n= 4); Group 2= the mice received AT-6 cream without imiquimod (IMQ) treatment for 5 days (n = 8); Group 3= the mice received vehicle with imiquimod treatment 5 days (n = 8); Group 4= mice received AT-6 cream with 4h after Imiquimod (IMQ) treatment for 5 days (n= 8); and Group 5= mice received AT-6 cream 1h before Imiquimod (IMQ) treatment for 5 days (n= 8).
FIG. 9: Immunostaining skin sections from mice's right ear. Groups 1 to 5 are as identified in FIG. 8.
FIG. 10: Bar-representation of the histological score for skin flanks (Y-axis) vs. the groups identified in FIG. 8 (X-axis).
FIG. 11: Bar-representation of the histological score for ear skins (Y-axis) vs. the groups identified in FIG. 8 (X-axis).
FIG. 12: Bar-representation of the number of mast cells counted/mm² in flank skin sections vs the groups identified in FIG. 8 (X-axis).
FIG. 13: Bar-representation of the number of mast cells counted/mm² in ear skin sections vs the groups identified in FIG. 8 (X-axis).
FIG. 14: Bar-representation of the number of the erythema index in flank skin sections vs. the groups identified in FIG. 8 (X-axis).

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

In a first aspect the present invention provides a combination comprising pea protein and a polysaccharide for use in the topical treatment or prevention of a skin inflammation disorder due to a skin barrier dysfunction.

The term "skin inflammation disorder due to a skin barrier dysfunction" is well-known for those people skilled in the art and encompasses those skin disorders caused by an alteration in any of the components forming the skin barrier. For illustrative non-limitative purposes, reference is made to Grainne M. O. et al., "Filaggrin in atopic dermatitis", J. Allergy Clin. Immunol., 2008, vol.122(4), 689-693; Dorota Purzycka-Bohdan et al., "Genetic background of skin barrier dysfunction in the pathogenesis of psoriasis vulgaris", Postepy Dermatol Alergol., 2015, 32(2), 123-126; and Flavia Alvim Sant'Anna Addor "Skin barrier in rosacea", An Bras Dermatol., 2016, vol. 91(1), 59-63, wherein skin barrier disfunction is analysed in disorders such as atopic dermatitis, psoriasis or rosacea.

In the present invention, the term "polysaccharide" refers to a polymeric carbohydrate molecule composed of long chains of monosaccharide units bound together by glycosidic linkages and on hydrolysis give the constituent monosaccharides or oligosaccharides. They range in structure from linear to highly branched. Examples include storage polysaccharides such as starch and glycogen, and structural polysaccharides such as cellulose and chitin. In the context of the invention, the polysaccharide is mucoadhesive, i.e., that it adheres on mucous membrane. There are well-known tests for measuring the mucoadhesion of a polysaccharide such as the one provided below, in the section of Examples, based on the work of mucoadhesion.

In one embodiment of the first aspect of the invention, optionally in combination with any of the embodiments provided below, the polysaccharide is xyloglucan, fucoidan or ulvan.

In the present invention, the term "xyloglucan" refers to a backbone of β1→4-linked glucose residues, most of which are substituted with 1-6 linked xylose sidechains. The xylose residues are often capped with a galactose residue sometimes followed by a fucose residue. Xyloglucan has the CAS number 37294-28-3.

A particularly rich source of xyloglucan is the seed of the tamarind (Tamarindus indica), a tropical tree from East Africa. Xyloglucans extracts from Tamarindus indica are available in the market from example from Indena (Italy) (Xilogel®), Megazyme, and from DSP Gokyo Food & Chemical (Japan) (Glyloid®).

In the present invention the term "fucoidan" refers to a non-gelling sulfated polysaccharide that have a backbone built of (1→3)-linked α-l-fucopyranosyl or of alternating (1→3)- and (1 → 4)-linked α-l-fucopyranosyl residues, but also include sulfated galactofucans with backbones built of (1→6)-β-d-galacto- and/or (1→2)-β-d-mannopyranosyl units with fucose or fuco-oligosaccharide branching, and/or glucuronic acid, xylose or glucose substitutions, found mainly in various species of brown algae and brown seaweed such as mozuku, kombu, Fucus vesiculosus (bladderwrack), *Undaria pinnatifida* (wakame), and hijiki (variant forms of fucoidan have also been found in animal species, including the sea cucumber). The fucoidan extract obtained from wacame is acetylated and rich in galactose. The fucoidan extract from *Fucus vesiculosus* is not acetylated but high in fucose.

In the present invention the term "ulvan" refers to a polysaccharide derived from *Ulva lactuca.* This polysaccharide has been deeply characterised in the state of the art (Audrey R. et al., "Structure and Functional Properties of Ulvan, a Polysaccharide from Green Seaweeds", 2007, American Chemical Society, 8(6), 1765-1774).

In the present invention, the term "pea protein" is the generic name given to any protein isolate obtained from yellow pea, Pisum sativum, seeds. "Pea protein" contains Legumin, which has some similar properties to Casein, and pea protein products are promoted as an alternative to whey protein. "Pea protein" is worldwide sold under different trademarks such as, Nutralys®, and P80X, among others. And it can also be prepared from pea cultivars by well-known routine methods, such as alkali extraction/isoelectric precipitation (AE-IP), salt extraction-dialysis (SE), and micellar precipitation (MP), among others.

In one embodiment of the first aspect of the invention, optionally in combination with any of the embodiments provided above or below, the combination is selected from a mixture comprising pea protein and a polysaccharide; and a pea protein-polysaccharide conjugated product.

In another embodiment of the first aspect of the invention, optionally in combination with any of the embodiments provided above or below, the combination is a mixture comprising pea protein and a polysaccharide. The mixture can be prepared merely mixing the ingredients.

In another embodiment of the first aspect of the invention, optionally in combination with any of the embodiments provided above or below, the combination is a pea protein-polysaccharide conjugated product.

In the present invention the term "pea protein-polysaccharide conjugated product" refers to a conjugate wherein the protein and the polysaccharide forms a single entity either due to interactions between the radicals present in the backbone of the protein and polysaccharide or due to the formation of bonds.

In another embodiment of the first aspect of the invention, optionally in combination with any of the embodiments provided above or below, the combination is a pea protein-polysaccharide conjugated product which is obtainable by a process comprising:
(a) preparing a mixture comprising pea protein, a polysaccharide, and an appropriate polar solvent; wherein:
   - the weight ratio between polysaccharide and pea protein is comprised from 20:80 to 60:40, and
   - the pH of the solution is comprised from 8.0 to 10.5; and
(b) performing a Maillard reaction by heating the solution resulting from step (a) at an appropriate temperature for the necessary period of time to conjugate the protein and the polysaccharide.

For purposes of the present invention, any ranges given include both the lower and the upper end-points of the range. Ranges given, such as temperatures, times, and the like, should be considered approximate, unless specifically stated.

Maillard reaction comprises three main stages:

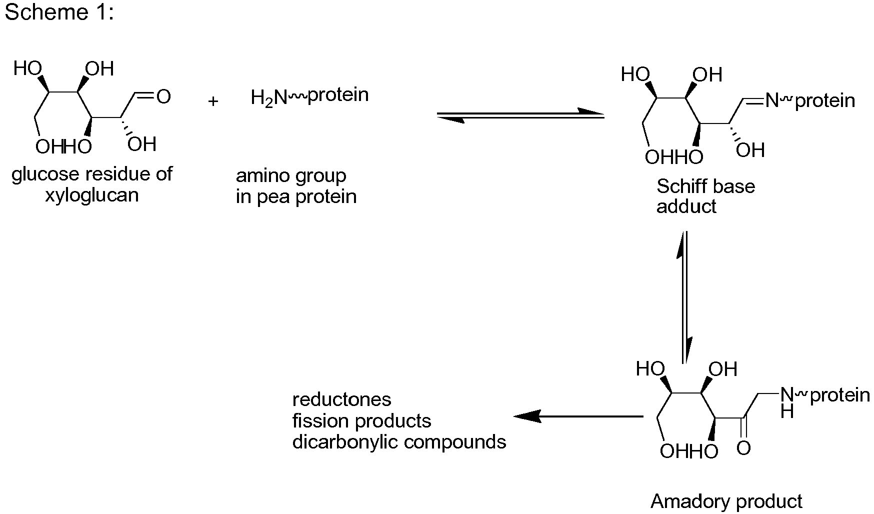

Briefly, (1) the carbonyl group on sugar (i.e., polysaccharide) reacts with a protein amino group by heating, thus producing a N-substituted glycosylamine; (2) the Schiff base adduct isomerases, giving Amadory product ketosamine. In a final step, Amadori product, under the specific reaction conditions of temperature and pH, can give rise to the formation of further products, such as reductones or fission products.

The term "weight ratio" refers to the relation of weights of polysaccharide:pea protein.

In one embodiment of the first aspect of the invention, optionally in combination with one or more of the embodiments provided above or below, the combination is a conjugated product obtainable by the process as defined above, wherein step (a) comprises the steps of:
(a.1) dissolving the pea protein in the appropriate polar solvent,
(a.2) adjusting the pH of the solution to a pH value comprised from 8.0 to 10.5, and
(a.3) adding the polysaccharide to the solution resulting from step (a.2);
or, alternatively,
(a.I) mixing the pea protein and the polysaccharide in the appropriate polar solvent, and
(a.II) adjusting the pH of the solution to a pH value comprised from 8.0 to 10.5;
or, alternatively,
(a.i) dissolving polysaccharide in the appropriate polar solvent,
(a.ii) dissolving pea protein in the appropriate polar solvent, and
(a.iii) mixing the solutions from steps (a.i) and (a.ii),
the adjustment of the pH being performed in step (a.ii), once dissolved the pea protein, or, alternatively, after step (a.iii), once solutions from steps (a.i) and (a.ii) are mixed.

In another embodiment of the first aspect of the invention, optionally in combination with one or more of the embodiments provided above or below, the combination is a conjugated product obtainable by the process as defined above, wherein step (a) is performed at room temperature.

In one embodiment of the first aspect of the invention, optionally in combination with one or more of the embodiments described above or below, the polar solvent is selected from the group consisting of: water, (C₁-C₆)alkyl OH, (C₁-C₆)alkyl-C(O)-(C₁-C₆)alkyl, (C₁-C₆)alkyl-C(O)H, dimethylformamide, and any mixture thereof. Examples of appropriate (C₃-C₆)cyclic ethers include tetrahydrofurane and dioxane. In another embodiment of the first aspect of the invention, optionally in combination with one or more embodiments provided above or below, the polar solvent is water.

The term (C₁-C₆) alkyl refers to a saturated straight or branched alkyl chain having from 1 to 4 carbon atoms. Illustrative non-limitative examples are: methyl, ethyl, propyl, isopropyl, butyl, isobutyl, sec-butyl, and tert-butyl.

In another embodiment of the first aspect of the invention, optionally in combination with one or more of the embodiments described above or below, the weight ratio polysaccharide:pea protein is comprised in the range from 25:75 to 55:45. In another embodiment of the first aspect of the invention, optionally in combination with one or more of the embodiments described above or below, the weight ratio polysaccharide:pea protein is comprised in the range from 30:70 to 50:50. In another embodiment of the first aspect of the invention, optionally in combination with one or more of the embodiments described above or below, the weight ratio polysaccharide:pea protein is 30:70.

In another embodiment of the first aspect of the invention, optionally in combination with one or more of the embodiments provided above or below, the combination is a conjugated product obtainable by the process as defined above, wherein step (a) is performed by: (a.1) dissolving the pea protein in water, (a.2) adjusting the pH of the solution to a pH value comprised from 8.0 to 10.5, and (a.3) adding the polysaccharide to the solution resulting from step (a.2); the weight ratio between polysaccharide and pea protein being comprised from 25:75 to 55:45.

In another embodiment of the first aspect of the invention, optionally in combination with one or more of the embodiments provided above or below, the combination is a conjugated product obtainable by the process as defined above, wherein step (a) is performed by: (a.1) dissolving the pea protein in water, (a.2) adjusting the pH of the solution to a pH value comprised from 8.0 to 10.5, and (a.3) adding the xyloglucan to the solution resulting from step (a.2); the weight ratio between xyloglucan and pea protein being comprised from 25:75 to 55:45.

In another embodiment of the first aspect of the invention, optionally in combination with one or more of the embodiments provided above or below, the combination is a conjugated product obtainable by the process as defined above, wherein step (a) is performed by: (a.1) dissolving the pea protein in water, (a.2) adjusting the pH of the solution to a pH value comprised from 8.0 to 10.5, and (a.3) adding the polysaccharide to the solution resulting from step (a.2); the weight ratio between polysaccharide and pea protein being 30:70.

In another embodiment of the first aspect of the invention, optionally in combination with one or more of the embodiments provided above or below, the combination is a conjugated product obtainable by the process as defined above, wherein step (a) is performed by: (a.1) dissolving the pea protein in water, (a.2) adjusting the pH of the solution to a pH value comprised from 8.0 to 10.5, and (a.3) adding the xyloglucan to the solution resulting from step (a.2); the weight ratio between xyloglucan and pea protein being 30:70.

In another embodiment of the first aspect of the invention, optionally in combination with one or more embodiments provided above or below, the combination is a conjugated product obtainable by the process as defined above, wherein the pH of the solution is adjusted to a value comprised from 9.5 to 10.5.

In another embodiment of the first aspect of the invention, optionally in combination with one or more embodiments provided above or below, the combination is a conjugated product obtainable by the process as defined above, wherein the pH of the solution is adjusted to 10.0.

In another embodiment of the first aspect of the invention, optionally in combination with one or more of the embodiments provided above or below, the combination is a conjugated product obtainable by the process as defined above, wherein step (a) is performed by: (a.1) dissolving the pea protein in water, (a.2) adjusting the pH of the solution to a pH value comprised from 9.5 to 10.5, and (a.3) adding the polysaccharide to the solution resulting from step (a.2); the weight ratio between polysaccharide and pea protein being comprised from 25:75 to 55:45.

In another embodiment of the first aspect of the invention, optionally in combination with one or more of the embodiments provided above or below, the combination is a conjugated product obtainable by the process as defined above, wherein step (a) is performed by: (a.1) dissolving the pea protein in water, (a.2) adjusting the pH of the solution to a pH value comprised from 9.5 to 10.5, and (a.3) adding the xyloglucan to the solution resulting from step (a.2); the weight ratio between xyloglucan and pea protein being comprised from 25:75 to 55:45.

In another embodiment of the first aspect of the invention, optionally in combination with one or more of the embodiments provided above or below, the combination is a conjugated product obtainable by the process as defined above, wherein step (a) is performed by: (a.1) dissolving the pea protein in water, (a.2) adjusting the pH of the solution to a pH value comprised from 9.5 to 10.5, and (a.3) adding the polysaccharide to the solution resulting from step (a.2); the weight ratio between polysaccharide and pea protein being 30:70.

In another embodiment of the first aspect of the invention, optionally in combination with one or more of the embodiments provided above or below, the combination is a conjugated product obtainable by the process as defined above, wherein step (a) is performed by: (a.1) dissolving the pea protein in water, (a.2) adjusting the pH of the solution to a pH value comprised from 9.5 to 10.5, and (a.3) adding the xyloglucan to the solution resulting from step (a.2); the weight ratio between xyloglucan and pea protein being 30:70.

In order to adjust the pH of the mixture protein, polysaccharide and polar solvent, any appropriate base can be added, such as an alkali metal or alkaline earth metal hydroxides. Illustrative non-limitative examples are NaOH, KOH, Ca(OH)₂, among others.

In another embodiment of the first aspect of the invention, optionally in combination with one or more embodiments provided above or below, the pH is adjusted to 10.0 by adding NaOH.

In another embodiment of the first aspect of the invention, optionally in combination with one or more embodiments provided above or below, the combination is a conjugated product obtainable by the process as defined above, wherein the weight ratio of polysaccharide:pea protein is 30:70, and the process for its obtaining comprises the steps of:
(i) mixing the pea protein with water;
(ii) adjusting the pH of the solution resulting from step (i) to a value of 9.5 to 10.5;
(iii) adding the polysaccharide to the solution resulting from step (ii); and
(iv) performing Maillard reaction by heating the solution resulting from step (iii) at a temperature comprised from 30 to 190 °C for the necessary period of time to conjugate the protein and the polysaccharide.

In another embodiment of the first aspect of the invention, optionally in combination with one or more embodiments provided above or below, the combination is a conjugated product obtainable by the process as defined above, wherein the weight ratio of xyloglucan:pea protein is 30:70, and the process for its obtaining comprises the steps of:
(i) mixing the pea protein with water;
(ii) adjusting the pH of the solution resulting from step (i) to a value of 9.5 to 10.5;
(iii) adding the xyloglucan to the solution resulting from step (ii); and
(iv) performing Maillard reaction by heating the solution resulting from step (iii) at a temperature comprised from 30 to 190 °C for the necessary period of time to conjugate the protein and the xyloglucan.

In another embodiment of the first aspect of the invention, optionally in combination with one or more embodiments provided above or below, the combination is a conjugated product obtainable by the process as defined above, wherein step (b) can be performed heating the mixture resulting from step (a) at a temperature comprised from 30 to 190 °C for the necessary period of time to obtain the conjugate. In another embodiment of the first aspect of the invention, optionally in combination with one or more embodiments provided above or below, the combination is a conjugated product obtainable by the process as defined above, wherein step (b) can be performed heating the mixture resulting from step (a) at a temperature comprised from 35 to 170 °C for the necessary period of time to obtain the conjugate. In another embodiment of the first aspect of the invention, optionally in combination with one or more embodiments provided above or below, the combination is a conjugated product obtainable by the process as defined above, wherein step (b) can be performed heating the mixture resulting from step (a) at a temperature comprised from 155 to 165 °C for the necessary period of time to obtain the conjugate. In another embodiment of the first aspect of the invention, optionally in combination with one or more embodiments provided above or below, the combination is a conjugated product obtainable by the process as defined above, wherein step (b) can be performed heating the mixture resulting from step (a) at a temperature of 160 °C for the necessary period of time to obtain the conjugate.

In another embodiment of the first aspect of the invention, optionally in combination with one or more embodiments provided above or below, the combination is a conjugated product obtainable by the process as defined above, wherein step (b) can be performed heating the mixture resulting from step (a) at a temperature comprised from 30 to 190 °C until dryness. In another embodiment of the first aspect of the invention, optionally in combination with one or more embodiments provided above or below, the combination is a conjugated product obtainable by the process as defined above, wherein step (b) can be performed heating the mixture resulting from step (a) at a temperature comprised from 35 to 170 °C until dryness. In another embodiment of the first aspect of the invention, optionally in combination with one or more embodiments provided above or below, the combination is a conjugated product obtainable by the process as defined above, wherein step (b) can be performed heating the mixture resulting from step (a) at a temperature comprised from 155 to 165 °C until dryness. In another embodiment of the first aspect of the invention, optionally in combination with one or more embodiments provided above or below, the combination is a conjugated product obtainable by the process as defined above, wherein step (b) can be performed heating the mixture resulting from step (a) at a temperature of 160 °C until dryness.

Step (b) can be performed heating the mixture resulting from step (a) in an apparatus such as an oven or an atomizer, among others. Depending on the apparatus used for performing step (b), the conditions of temperature and time can be different.

Optionally, once the pH of the mixture has been adjusted and prior to step (b) (Maillard reaction), the mixture can be lyophilized. Performing Maillard reaction with the alkaline mixture previously lyophilized can improve the cross-linking efficiency between protein and polysaccharide.

In one embodiment of the first aspect of the invention, the combination is a conjugated product obtainable by the process comprising the steps of: (a) preparing a mixture comprising pea protein, polysaccharide and water, the weight ratio polysaccharide:pea protein being 30:70, and the pH of the solution being comprised from 9.5 to 10.5, and (b) performing Maillard reaction by heating the solution resulting from step (a) at a temperature comprised from 35 to 190 °C.

In one embodiment of the first aspect of the invention, the combination is a conjugated product obtainable by the process comprising the steps of: (a) preparing a mixture comprising pea protein, xyloglucan and water, the weight ratio between xyloglucan and pea protein being 30:70, and the pH of the solution being comprised from 9.5 to 10.5, and (b) performing Maillard reaction by heating the solution resulting from step (a) at a temperature comprised from 35 to 190 °C.

In one embodiment of the first aspect of the invention, the combination is a conjugated product obtainable by the process comprising the steps of: (a) preparing a mixture comprising pea protein, polysaccharide, and water, the weight ratio between polysaccharide and pea protein being 30:70, and the pH of the solution is 10, and (b) performing Maillard reaction by heating the solution resulting from step (a) at a temperature comprised from 35 to 190 °C.

In one embodiment of the first aspect of the invention, the combination is a conjugated product obtainable by the process comprising the steps of: (a) preparing a mixture comprising pea protein, xyloglucan, and water, the weight ratio between xyloglucan and pea protein being 30:70, and the pH of the solution is 10, and (b) performing Maillard reaction by heating the solution resulting from step (a) at a temperature comprised from 35 to 190 °C.

In one embodiment of the first aspect of the invention, the combination is a conjugated product obtainable by the process comprising the steps of: (a) preparing a mixture comprising pea protein, polysaccharide, and water, the weight ratio between polysaccharide and pea protein being 30:70, and the pH of the solution being comprised from 9.5 to 10.5, and (b) performing Maillard reaction by heating the solution resulting from step (a) at a temperature comprised from 155 to 165 °C.

In one embodiment of the first aspect of the invention, the combination is a conjugated product obtainable by the process comprising the steps of: (a) preparing a mixture comprising pea protein, xyloglucan, and water, the weight ratio between xyloglucan and pea protein being 30:70, and the pH of the solution being comprised from 9.5 to 10.5, and (b) performing Maillard reaction by heating the solution resulting from step (a) at a temperature comprised from 155 to 165 °C.

In one embodiment of the first aspect of the invention, the combination is a conjugated product obtainable by the process comprising the steps of: (a) preparing a mixture comprising pea protein, polysaccharide, and water, the weight ratio between polysaccharide and pea protein being 30:70 and the pH of the solution being 10, and (b) performing Maillard reaction by heating the solution resulting from step (a) at a temperature comprised from 155 to 165 °C.

In one embodiment of the first aspect of the invention, the combination is a conjugated product obtainable by the process comprising the steps of: (a) preparing a mixture comprising pea protein, xyloglucan, and water, the weight ratio between xyloglucan and pea protein being 30:70 and the pH of the solution being 10, and (b) performing Maillard reaction by heating the solution resulting from step (a) at a temperature comprised from 155 to 165 °C.

In one embodiment of the first aspect of the invention, the combination is a conjugated product obtainable by the process comprising the steps of: (a) preparing a mixture comprising pea protein, polysaccharide, and water, the weight ratio between polysaccharide and pea protein being 30:70, and the pH of the solution being comprised from 9.5 to 10.5, and (b) performing Maillard reaction by heating the solution resulting from step (a) at a temperature of 160 °C.

In one embodiment of the first aspect of the invention, the combination is a conjugated product obtainable by the process comprising the steps of: (a) preparing a mixture comprising pea protein, xyloglucan, and water, the weight ratio between xyloglucan and pea protein being 30:70, and the pH of the solution being comprised from 9.5 to 10.5, and (b) performing Maillard reaction by heating the solution resulting from step (a) at a temperature of 160 °C.

In one embodiment of the first aspect of the invention, the combination is a conjugated product obtainable by the process comprising the steps of: (a) preparing a mixture comprising pea protein, polysaccharide, and water, the weight ratio between polysaccharide and pea protein being 30:70, and the pH of the solution being 10, and (b) performing Maillard reaction by heating the solution resulting from step (a) at a temperature of 160 °C.

In one embodiment of the first aspect of the invention, the combination is a conjugated product obtainable by the process comprising the steps of: (a) preparing a mixture comprising pea protein, xyloglucan, and water, the weight ratio between xyloglucan and pea protein being 30:70, and the pH of the solution being 10, and (b) performing Maillard reaction by heating the solution resulting from step (a) at a temperature of 160 °C.

In one embodiment of the first aspect of the invention, optionally in combination with any of the embodiments provided above or below, the skin disorder is an inflammatory skin disorder.

In another embodiment of the first aspect of the invention, optionally in combination with any of the embodiments provided above or below, the combination is for use in the prophylaxis and/or treatment of an inflammatory skin disorder which is selected from atopic dermatitis, psoriasis, rosacea and skin lesions due to radio- or chemotherapy.

In another embodiment of the first aspect of the invention, optionally in combination with any of the embodiments provided above or below, the combination is for use in the prophylaxis and/or treatment of atopic dermatitis.

Also disclosed is a topical pharmaceutical or cosmetic composition comprising: (i) an effective amount of a combination of pea protein and polysaccharide; and (ii) one or more appropriate topical pharmaceutically or cosmetically acceptable excipients or carriers. The invention is defined by the claims. Any subject-matter falling outside the scope of the claims is provided for information purposes only.

The term "topical" refers to the application of the pharmaceutical to the skin or mucous membrane. Thus, the topical composition of the second aspect of the invention can be applied, for example, on skin, nasal mucosa, oral mucosa or vaginal mucosa, among others.

All the embodiments provided above as "embodiments of the first aspect of the invention" concerning the combination comprising pea protein and polysaccharide are also embodiments of the combination referred in the compositions of the second aspect of the present invention.

An "effective amount" of the combination refers to the amount of combination that provides a therapeutic or cosmetic effect after its application. In one embodiment of the second aspect of the invention, optionally in combination with any of the embodiments provided above or below, the combination comprising pea protein and polysaccharide is at a percentage by weight, with respect to the total weight of the pharmaceutical or cosmetic composition, comprised from 0.5 to 20 %. In one embodiment of the second aspect of the invention, optionally in combination with any of the embodiments provided above or below, the combination comprising pea protein and polysaccharide is at a percentage by weight, with respect to the total weight of the pharmaceutical or cosmetic composition, comprised from 1.0 to 10 %. In one embodiment of the second aspect of the invention, optionally in combination with any of the embodiments provided above or below, the combination comprising pea protein and polysaccharide is at a percentage by weight, with respect to the total weight of the pharmaceutical or cosmetic composition, comprised from 1.0 to 6%.

The term "pharmaceutically acceptable" refers to the excipients or carriers appropriate for use in pharmaceutical technology, for the preparation of compositions for medical use.

The term "cosmetically acceptable" refers to excipients or carriers that are appropriate for use in contact with human skin or mucosa without inappropriate allergic response, instability, incompatibility, or toxicity among others.

In a particular embodiment, the topical composition is a pharmaceutical composition comprising an effective amount of the combination as defined above together with one or more appropriate topical pharmaceutically acceptable excipients or carriers.

In another embodiment of the second aspect of the invention, optionally in combination with any of the embodiments provided above or below, the topical composition can comprise other active ingredients, such as sucralfate.

The topical compositions defined above comprise appropriate excipients or carriers for topical administration that can be pharmaceutical or cosmetic excipients, including, but not limited to, repairing cutaneous barrier function agent, a hydrating agent, an emollient, an emulsifier, a thickener, a humectant, a pH-regulating agent, an antioxidant, a preservative agent, a vehicle, or their mixtures. The excipients or carriers used have affinity for the skin, are well tolerated, stable, and are used in an amount adequate to provide the desired consistency, and ease application.

Examples of appropriate topical skin barrier recovery agent include, but are not limited to, ceramides, cholesterol, fatty acids, and precursors of these lipids including cerebrosides, sphingoid bases such as phytosphingosine or sphingosine, or phospholipids including phosphatidylcholine, and agents that promote the synthesis of epidermal lipids like urea, dexpanthenol, and alpha-hydroxyacids including lactic acid among others.

Examples of appropriate topical hydrating agent include, but are not limited to, collagen, collagen amino acids, dimethiconol, glycine, hyaluronic acid, dimethylsilanol hyaluronate, magnesium stearate, maltitol, maltose, pyrrolidone carboxylic acid (PCA), manganese PCA, sodium PCA, mannitol, trehalose, trilactin, glucose, glutamic acid, hydrolyzed caesalpinia spinosa gum, caesalpinia spinosa gum, prunus persica extract, prunus serotina extract, echinacea angustifolia extract, Echinacea purpurea extract, methyl gluceth, hydrolyzed wheat gluten, erythritol, aluminium stearoyl glutamate, copper acetylmethionate, or ditridecyl dimmer dilinoleate. Preferably the hydrating agent is selected from the group consisting of glucose, glycine, lysine, glutamic acid, hydrolyzed caesalpinia spinosa gum, caesalpinia spinosa gum, sodium PCA, and their mixtures.

Examples of appropriate topical emollient agents include, but are not limited to, octyl hydroxystearate, lanolin, caprylic/capric triglyceride, cetyl palmitate, octyldodecanol, cetyl alcohol, isopropyl isostearate, glyceryl dilaurate, isopropyl myristate, palm alcohol, dimethicone, squalane, plukenetia volubilis seed oil, butyrospermum parkii butter, sucrose cocoate, or their mixtures. Preferably the emollient is selected from the group consisting of dimethicone, squalane, plukenetia volubilis seed oil, butyrospermum parkii butter, caprylic/capric triglyceride, octyldodecanol, or their mixtures.

Examples of appropriate emulsifier include, but are not limited to, glyceryl trioleate, glyceryl oleate, acetylated sucrose distearate, sorbitan trioleate, polyoxyethylene monostearate, glycerol monooleate, sucrose distearate, polyethylene glycol monostearate, octyl phenoxypoly (ethyleneoxy) ethanol, deacylerin penta-isostearate, sorbitan sesquioleate, hydroxylated lanolin, lecithin, lanolin, triglyceryl diisostearate, polyoxyethylene oleyl ether, calcium stearoyl-2-lactylate, sodium lauroyl lactylate, sodium stearoyl lactylate, cetearyl glucoside, methyl glucoside sesquistearate, sorbitan monopalmitate, methoxy polyethylene glycol-22/dodecyl glycol copolymer, polyethylene glycol-45/dodecyl glycol copolymer, polyethylene glycol 400 distearate and glyceryl stearate, candelilla/jojoba/rice bran polyglyceryl-3 esters, cetyl phosphate, potassium cetyl phosphate, or their mixtures. Preferably, the emulsifier is selected group consisting of glyceryl oleate, lecithin, sodium lauroyl lactylate, sodium stearoyl lactylate, glyceryl stearate, candelilla/jojoba/rice bran polyglyceryl-3 esters, and their mixtures.

Examples of appropriate surfactant agents include, but are not limited to, non-ionic, ionic (either anionic or cationic) or zwitterionic (or amphoteric wherein the head of the surfactant contains two oppositely charged groups) surfactants. Examples of anionic surfactants include, but are not limited to, those based on sulfate, sulfonate or carboxylate anions such as perfluorooctanoate (PFOA or PFO), alkyl benzene sulfonate, soaps, fatty acid salts, or alkyl sulfate salts such as perfluorooctanesulfonate (PFOS), sodium dodecyl sulfate (SDS), ammonium lauryl sulfate, or sodium lauryl ether sulfate (SLES). Examples of cationic surfactants include, but are not limited to, those based on quaternary ammonium cations such as or alkyltrimethylammonium including cetyl trimethylammonium bromide (CTAB) a.k.a., or hexadecyl trimethyl ammonium bromide, cetylpyridinium chloride (CPC), polyethoxylated tallow amine (POEA), benzalkonium chloride (BAC), or benzethonium chloride (BZT). Examples of zwitterionic surfactants include, but are not limited to dodecyl betaine, cocamidopropyl betaine, or coco ampho glycinate. Examples of non-ionic surfactants include, but are not limited to, alkyl poly(ethylene oxide), alkylphenol poly(ethylene oxide), copolymers of poly(ethylene oxide), poly(propylene oxide) (commercially called Poloxamers or Poloxamines), alkyl polyglucosides including octyl glucoside and decyl maltoside, fatty alcohols including cetyl alcohol and oleyl alcohol, cocamide MEA, cocamide DEA, or polysorbates including tween 20, tween 80, or dodecyl dimethylamine oxide. Preferably, the surfactant is foaming and skin friendly, including polysorbate 20 or 40, coco glucoside, lauryl glucoside, decyl glucoside, lauryl sulfates such as ammonium, sodium, magnesium, MEA, triethylamine (TEA), or mipa lauryl sulfate, cocamidopropyl betain, or sodium alkyl sulfosuccinates.

Examples of appropriate topical humectants include, but are not limited to, glycerin, diglycerin, ethylhexylglycerin, glucose, honey, lactic acid, polyethylene glycol, propylene glycol, sorbitol, sucrose, or threalose. Preferably, the humectant is selected group consisting of glycerin, diglycerin, ethylhexylglycerin, and their mixtures.

Examples of appropriate topical pH-regulating agents include, but are not limited to, acetic acid, lactic acid, citric acid, ethanolamine, formic acid, oxalic acid, potassium hydroxide, sodium hydroxide, triethanolamine, or their mixtures. Preferably, the pH-regulating agent is selected group consisting of triethanolamine, sodium hydroxide, lactic acid, and citric acid.

Examples of appropriate antioxidants include, but are not limited to, free radical scavengers or reducing agents such as, acetyl cysteine, ascorbic acid, ascorbyl palmitate, butylated hydroxytoluene, green tea extract, caffeic acid, cysteine, tocopherol, ubiquinone, propyl gallate, butylated hydroxytoluene (BHT), and their mixtures. Preferably, the antioxidant agent is selected group consisting of ascorbyl palmitate, and tocopherol.

Examples of appropriate preservative agents include, but are not limited to, benzoic acid, butylparaben, ethylparaben, propylparaben, methylparaben, sorbic acid, potassium sorbate, sodium benzoate, phenoxyethanol, triclosan, or their mixtures. Preferably, the preservative agent is selected group consisting of potassium sorbate, sodium benzoate, and phenoxyethanol.

Examples of appropriate viscosity agents include, but are not limited to, cellulose or their derivatives such as hydroxypropyl methylcellulose,
polyethylene glycol, microcrystalline cellulose, cetearyl alcohol, alginates, branched polysaccharides, fumed silica, xanthan gum, carbomer, and polyacrylates. Preferably, the viscosity agent is selected group consisting of microcrystalline cellulose, cetearyl alcohol, cellulose, xanthan gum, and carbomer.

The compositions mentioned above also include a vehicle. Examples of vehicles include, but are not limited to, water, propylene glycol, butylene glycol, ethanol, isopropanol, or silicones. Preferably, the vehicle is water.

Additionally, the compositions of the present invention may contain other ingredients, such as fragrances, colorants, and other components known in the state of the art for use in topical formulations.

The topical compositions of the invention can be formulated in several forms that include, but are not limited to, solutions, aerosols and non-aerosol sprays, shaving creams, powders, mousses, lotions, gels, sticks, ointments, pastes, creams, shampoos, shower gel, body washes or face washes.

Another topical composition used is formulated preferably as a "surfactant base". A surfactant base is a blend of at least two surfactants. Surfactants are commonly used in cleaning products, breaking up stains and keeping the dirt in the water solution to prevent its re-deposition onto the surface. Surfactants disperse dirt that normally does not dissolve in water, becoming it dispersible in water, and removable with the wash water. The above mentioned surfactants are included to lower the surface tension.

Topical compositions of the present invention can be prepared according to methods well known in the state of the art. The appropriate excipients and/or carriers, and their amounts, can readily be determined by those skilled in the art according to the type of formulation being prepared.

In one embodiment of the second aspect of the invention, optionally in combination with any of the embodiments provided above or below, the topical composition is in the form of a patch.

The patch comprises a carrier material, the combination as defined in the first aspect of the invention, and one or more appropriate topical pharmaceutically or cosmetically acceptable excipients or carriers.

The term "carrier material" has to be understood as a substrate of suitable material allowing depositing the composition of the invention thereon, its transport and its release at the desired site, for example, in the site where the compositions of the invention has to exert its effect. Said carrier material can be a solid support. Illustrative, non-limiting examples of solid supports include dressings, band-aids, compresses, plasters, etc. Illustrative, non-limiting examples of liquid supports include gels, sprays, mouthwashes, etc. The interaction between the components of the composition and the solid material can be a physical or chemical interaction.

The skilled person, using the general knowledge, is able to select on the basis of the carrier material, the appropriate amount of the combination, and excipients or carriers as well the appropriate protocol to apply it to the carrier material.

In another embodiment of the second aspect of the invention, optionally in combination with any of the embodiments provided above or below, the topical composition is in the form of an impregnated (the carrier material is cotton and the composition is embedded within), or medicated (the carrier material is a plastic or a non-woven fabric and the composition is applied on or incorporated in) patch.

In a third aspect the present invention provides a topical medical device comprising the topical pharmaceutical composition for use as defined by the claims.

All the embodiments provided above for the topical composition of the second aspect of the invention, are also embodiments of the topical composition referred in the third aspect of the invention.

In one embodimen to of the third aspect of the invention, the topical composition is applied on a carrier material which as defined above in the second aspect of the invention.

In another embodiment of the third aspect of the invention, optionally in combination with any of the embodiments provided above or below, the topical medical device is a patch. In still another embodiment, optionally in combination with any of the embodiments provided above or below, the patch is an impregnated, medicated or bis-medicated patch.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

### Examples

Pea protein was purchased from Roquette Frères and xyloglucan was purchased from DSP Gokyo.

### 1. Conjugated xyloglucan:pea protein 30:70 (hereinafter also referred as "AT-6")

14 g of pea protein were dissolved in 500 mL of water (this was the amount of water needed to completely dissolve the weighted pea powder). Next, the pH of the solution was adjusted to 10 by the addition of NaOH. Once the pH was adjusted, 6 g of xyloglucan were added to the mixture and more water was added until the total final weight of the solution was 1000 g. The mixture was homogenized using ultra-turrax T25 basic (IKA-WERKE GMBH &CO KG D-79219 Stanfer, Germany).

The whole preparation process was performed at room temperature.

The resulting mixture was atomized in a Mini Spray Dryer B-290, BÜCHI Labortechnik AG (Flawil, Switzerland) at 160°C until a dry powder was obtained.

### - Characterization of conjugated xyloglucan:protein (AT-6)

A complexation study was performed with "AT6" by H-NMR to confirm the covalent bond between pea protein and xyloglucan. Technology DOSY allows analyzing mixtures of compounds by dividing the resonances of compounds with different diffusion coefficients. The spectrum presents a horizontal axis (T2) that identifies the resonance frequencies of the proton (ppm) and a vertical axis that presenting the diffusion parameter.

Three solutions were prepared: one with protein alone, one with xyloglucan alone, and a third one with AT-6. Samples were prepared as follows: 10 mg of either protein, xyloglucan or AT-6 were suspended in 10 mL D₂O and kept
under stirring for 48 hours. The resulting suspension was then filtered, and the undissolved material was discarded.

The resulting solutions were subjected to DOSY. NMR spectra were recorded at 298 K in D₂O on a Varian 500 MHz instrument equipped with a pulse-field gradient probe. The HDO residual solvent peak (δ = 4.65 ppm) was used as an internal standard. 1H NMR spectra were recorded using solvent suppression pulse sequences (WET). Diffusion-ordered NMR spectroscopy (DOSY) studies were performed using a DgcsteSL pulse sequence, optimizing experimental parameters according to the sample under investigation. Diffusion gradients were progressively incremented over 30 steps, varying the gradient strength from 1.8 to 50.0 gauss/cm. 16 Transients were acquired for each increment, with a diffusion-gradient length of 4 ms and diffusion delays of 400 ms.

The DOSY NMR analysis on the soluble fractions of the three samples gave the following results, as far as the self-diffusion coefficient ranges are concerned:
Sample A (Pea): 1-3x10⁻¹⁰ (m² s⁻¹)
Sample B (Tamarind): 0.08 - 0.1x10⁻¹⁰ (m² s⁻¹)
Sample C (Pea+Tamarind): 0.1 - 0.3x10⁻¹⁰ (m² s⁻¹)

These results indicated that the Maillard reaction between a polysaccharide (xyloglucan) and protein (pea protein) provides a conjugate wherein protein and polysaccharide are covalently bound.

### 2. Example of topical compositions of the invention

### 2.1. Cream comprising a combination of pea protein and xyloglucan

| Description | % (weight/weight) |
|---|---|
| AQUA | 72,83 |
| C12-15 ALKYL BENZOATE | 4,21 |
| OCTYLDODECANOL | 3,16 |
| ISONONYL ISONONANOATE | 2,1 |
| GLYCERIN | 2,1 |
| CAPRYLIC/CAPRIC TRIGLYCERIDE | 2,1 |
| CERA ALBA | 2,1 |
| POLYGLYCERYL-10 STEARATE | 1,47 |
| POLYGLYCERYL-6 TRISTEARATE | 1,26 |
| CETEARYL ALCOHOL | 1,05 |
| CAPRYLYL GLYCOL | 0,74 |
| HYDROGENATED POLYISOBUTENE | 0,53 |
| HYDROXYPROPYL GUAR | 0,42 |
| BISABOLOL | 0,21 |
| PANTHENOL | 0,21 |
| GLYCERYL CAPRYLATE | 0,21 |
| VACCINIUM MYRTILLUS SEED OIL | 0,1 |
| DISODIUM EDTA | 0,1 |
| ETHYLHEXYLGLYCERIN | 0,1 |
| AT/6 | 5 |

### 2.2. Gel comprising a combination of pea protein and xyloglucan

| Description | % (weight/weight) |
|---|---|
| AQUA | 96,67 |
| PHENOXYETHANOL | 0,81 |
| AMMONIUM ACRYLOYLDIMETHYLTAURATE/VP COPOLYMER | 0,51 |
| IMIDAZOLIDINYL UREA | 0,21 |
| GLYCERIN | 0,2 |
| BISABOLOL | 0,1 |
| PANTHENOL | 0,1 |
| AT/6 | 1,5 |

### 2.3. Lotion comprising a combination of pea protein and xyloglucan

| Description | % (weight/weight) |
|---|---|
| AQUA | 76,83 |
| C12-15 ALKYL BENZOATE | 5,15 |
| OCTYLDODECANOL | 3,09 |
| GLYCERIN | 2,06 |
| CAPRYLIC/CAPRIC TRIGLYCERIDE | 2,06 |
| ISONONYL ISONONANOATE | 2,06 |
| POLYGLYCERYL-10 STEARATE | 1,08 |
| POLYGLYCERYL-6 TRISTEARATE | 0,93 |
| CETEARYL ALCOHOL | 0,77 |
| CAPRYLYL GLYCOL | 0,72 |
| PANTHENOL | 0,51 |
| BISABOLOL | 0,51 |
| HYDROGENATED POLYISOBUTENE | 0,51 |
| HYDROXYPROPYL GUAR | 0,31 |
| GLYCERYL CAPRYLATE | 0,21 |
| DISODIUM EDTA | 0,1 |
| ETHYLHEXYLGLYCERIN | 0,1 |
| AT/6 | 3 |

### 2.4. Cream comprising comprising a combination of pea protein and xyloglucan, and fucoidans

| Description | % (weight/weight) |
|---|---|
| AQUA | 71,83 |
| C12-15 ALKYL BENZOATE | 4,21 |
| OCTYLDODECANOL | 3,16 |
| ISONONYL ISONONANOATE | 2,1 |
| GLYCERIN | 2,1 |
| CAPRYLIC/CAPRIC TRIGLYCERIDE | 2,1 |
| CERA ALBA | 2,1 |
| POLYGLYCERYL-10 STEARATE | 1,47 |
| POLYGLYCERYL-6 TRISTEARATE | 1,26 |
| CETEARYL ALCOHOL | 1,05 |
| CAPRYLYL GLYCOL | 0,74 |
| HYDROGENATED POLYISOBUTENE | 0,53 |
| HYDROXYPROPYL GUAR | 0,42 |
| BISABOLOL | 0,21 |
| PANTHENOL | 0,21 |
| GLYCERYL CAPRYLATE | 0,21 |
| VACCINIUM MYRTILLUS SEED OIL | 0,1 |
| DISODIUM EDTA | 0,1 |
| ETHYLHEXYLGLYCERIN | 0,1 |
| AT/6 | 5,0 |
| MARITECH® UNDARIA PINNATIFIDA EXTRACT | 1,0 |

### 2.5. Gel comprising comprising a combination of pea protein and xyloglucan, and fucoidans

| Description | % (weight/weight) |
|---|---|
| AQUA | 95,67 |
| PHENOXYETHANOL | 0,81 |
| AMMONIUM ACRYLOYLDIMETHYLTAURATE/VP COPOLYMER | 0,51 |
| IMIDAZOLIDINYL UREA | 0,21 |
| GLYCERIN | 0,2 |
| BISABOLOL | 0,1 |
| PANTHENOL | 0,1 |
| AT/6 | 1,5 |
| MARITECH® UNDARIA PINNATIFIDA EXTRACT | 1,0 |

### 2.6. Lotion comprising a combination of pea protein and xyloglucan, and fucoidans

| Description | % by weight |
|---|---|
| AQUA | 75,83 |
| C12-15 ALKYL BENZOATE | 5,15 |
| OCTYLDODECANOL | 3,09 |
| GLYCERIN | 2,06 |
| CAPRYLIC/CAPRIC TRIGLYCERIDE | 2,06 |
| ISONONYL ISONONANOATE | 2,06 |
| POLYGLYCERYL-10 STEARATE | 1,08 |
| POLYGLYCERYL-6 TRISTEARATE | 0,93 |
| CETEARYL ALCOHOL | 0,77 |
| CAPRYLYL GLYCOL | 0,72 |
| PANTHENOL | 0,51 |
| BISABOLOL | 0,51 |
| HYDROGENATED POLYISOBUTENE | 0,51 |
| HYDROXYPROPYL GUAR | 0,31 |
| GLYCERYL CAPRYLATE | 0,21 |
| DISODIUM EDTA | 0,1 |
| ETHYLHEXYLGLYCERIN | 0,1 |
| AT/6 | 3,0 |
| MARITECH® UNDARIA PINNATIFIDA EXTRACT | 1,0 |

### 3. Efficacy on skin inflammation disorders

### 3.1. Materials and Methods

### - Animals

Specific pathogen-free 5-week-old female SKH-1 hairless mice (Envigo, Milan, Italy) were housed in a controlled environment (22 ± 2 °C, 55 ± 15% relative humidity, 12 h light/dark cycle). After a one-week acclimation, mice were fed a standard diet and water.

Animal experiments are in compliance with Italian regulations on protection of animals used for experimental and other scientific purposes (DM 116192) as well as EU regulations (OJ of EC L358/1 12/18/1986).

### - Histological and immunohistochemical analyses

Skin lesions, tight junctions and filaggrin (TJs) were fixed with 10% neutral formalin, embedded in paraffin, and sectioned at 5 µm.

The determination of the histological score was supported by a trained pathologist ignorant of the treatment status of the animals. Skin regions sections were stained with hematoxilin and eosin (HE). Inflammation was scored as follows: (0) infiltration of few inflammatory cells into dermis, (2) major infiltration of inflammatory cells into the dermis and (3) infiltration of the epidermis. Alterations of skin architecture were scored as follows: (0) no alteration, (1) epithelial hyperplasia, (0) no fibrosis, (1) fibrosis. The resulting score parameters were added in a total histological score ranging from 0 (healthy) to 5 (maximal histological damage) (Nolte *et al.,* 2013).

To detect infiltration of inflammatory cells including mast cells, sections were stained with toluidine blue, respectively.

ZO-1 and occludin expression were detected by polyclonal rabbit anti ZO-1 (1:100 Santa Cruz Biotechnology, Santa Cruz, CA, USA) and anti-occludin (1:100 Santa Cruz Biotechnology, Santa Cruz, CA, USA) following manufacturer's instructions.

Filaggrin expression was detected using polyclonal rabbit anti-filaggrin (Abcam, Edinburgh, UK) as a primary antibody at 1:200 overnight at 4 °C and secondary antibody (Santa Cruz Biotechnology, Santa Cruz, CA, USA) for 1 h, following manufacturer's instructions.

For all the primary antibodies, secondary antibody (Santa Cruz Biotechnology, Santa Cruz, CA, USA) was incubated for 1 h.

All stained sections were observed by an Inverted Microscope with twin CCD cameras (magnification, ×200; Nikon, Tokyo, Japan).

### - Quantitative morphology

The number of mast cells in toluidine blue-stained sections was measured from more than 10 fields in each sample using AxioVision software (CarlZeiss Vision, Jena, Germany).

Data were reported as the mean with standard deviation (SD).

### - Processing of lymph nodes

Animals were euthanized and body weights recorded. The draining (auricular) lymph nodes were excised, and were mechanically disaggregated and resuspended in a sterile saline solution containing 0.9% NaCl.

The incorporation of 5-bromo-2'-deoxyuridine and 5-fluoro-2'-deoxyuridine (BrdU, Sigma-Aldrich) in the cells was measured by using the ELISA kit (Cell proliferation ELISA, BrdU (colorimetric) version 13.0 cat no 11647229001), and recorded as average of BLI (BrdU labeling index)/n mice treated with the sample, with the positive control, and with negative control. Each extract was loaded and processed in triplicate by ELISA KIT. For each sample a Stimulation Index (SI) was obtained, relative to the vehicle.

### 3.2. Efficacy in atopic dermatitis models

### 3.2.1 Induction of AD-like skin lesions and bacterial infection, and sample treatment

Mice were divided into the following experimental groups:
Group 1 mice received vehicle (polysorbate 80) without oxazolone treatment for 4 weeks (n = 4);
Group 2 mice received AT6 cream 5% (with the composition provided in section 2.1. above) without oxazolone treatment for 4 weeks (n = 8);
Group 3 mice received AT6 gel 1,5% (with the composition provided in section 2.2.above) without oxazolone treatment for 4 weeks (n = 8);
Group 4 mice received AT6 lotion 3% (with the composition provided in section 2.3. above) without oxazolone treatment for 4 weeks (n = 8);
Group 5 mice received vehicle (polysorbate 80) with oxazolone treatment for 4 weeks (n = 8);
Group 6 mice received AT6 cream 5% (with the composition provided in section 2.1. above) with 1 hr before oxazolone treatment for 4 weeks (n= 8);
Group 7 mice received AT6 gel 1,5% (with the composition provided in section 2.2. above) with 1 hr before oxazolone treatment for 4 weeks (n= 8);
Group 8 mice received AT6 lotion 3% (with the composition provided in section 2.3. above) with 1 hr before oxazolone treatment for 4 weeks (n= 8);
Group 9: mice received AT6 cream 5% (with the composition provided in section 2.1. above) after oxazolone treatment for 2 weeks (n = 8);
Group 10 mice received AT6 gel 1,5% (with the composition provided in section 2.2. above) after oxazolone treatment for 2 weeks (n = 8);
Group 11 mice received AT6 lotion 3% (with the composition provided in section 2.3. above) after oxazolone treatment for 2 weeks (n = 8);
Group 12 mice received subcutaneous inoculation of 50 µl of *Staphylococcus aureus* bacterial suspension at a concentration of 1.5 x 10⁷ CFU/50 µl (skin infection) in addition to oxazolone for 2 weeks (n=8);
Group 13 mice received AT6 cream 5% (with the composition provided in section 2.1. above) 24 hrs after subcutaneous inoculation of Staphylococcus aureus bacterial suspension at a concentration of 1.5 x 10⁷ CFU/50 µl (skin infection) in addition to oxazolone for 2 weeks (n=8);
Group 14 mice received AT6 gel 1,5% (with the composition provided in section 2.2. above) 24 hrs after subcutaneous inoculation of *Staphylococcus aureus* bacterial suspension at a concentration of 1.5 x 10⁷ CFU/50 µl (skin infection) in addition to oxazolone for 2 weeks (n=8);
Group 15 mice received AT6 lotion 3% (with the composition provided in section 2.3. above)24 hrs after subcutaneous inoculation of *Staphylococcus aureus* bacterial suspension at a concentration of 1.5 x 10⁷ CFU/50 µl (skin infection) in addition to oxazolone for 2 weeks (n=8); and
Group 16: mice received hydrocortisone (HC; 2.5 mg/mice topically administered) before oxazolone treatment for 4 weeks (n = 8).

The amount of the composition of the invention applied is relative to the one necessary to cover the back of mice until the creation of a layer.

Mice, except those in groups 1-4, were topically treated with 200 µl of 0.5% oxazolone (Sigma- Aldrich, St. Louis, MO, USA) to the dorsal skin three times a week for 2 weeks (total of 12 challenges). Mice in groups 9-11 were topically treated with the topical compositions 2 weeks after applying oxazolone.

Group 16 HC daily for 4 weeks using oral feeding needles before the application of oxazolone treatment. At the end of the 4-week oral administration period, erythema index was measured according to Kim et al., 2014.

After the experiments, spleens and skin lesions were removed and stored at -80 °C until analysis.

Lymph nodes were removed from mice in groups 2-4 to verify the safety of topical compositions, in particular mice were injected with a solution of BrdU (Sigma-Aldrich) 10 mg/ml intraperitoneally 24h before sacrifice. Twenty-four hours post-injection, mice were sacrificed and the auricular lymph nodes were removed for ELISA kit and BrdU immunohistochemical detection by using anti-BrdU polyclonal rabbit antibody (Santa Cruz, CA, USA).

### 3.2.2. Results

### A) Histological evaluation

Histological examination of skin revealed characteristic pathological changes after oxazolone treatment compared to sham group.

Histological examination of skin revealed characteristic pathological changes after oxazolone treatment. The topical administration of AT-6 significantly reduces the degree of tissue injury (FIG. 1, A).

This was further supported by determining the histological score. FIG. 1 (B) shows that the topical administration of AT-6 (a combination of pea protein and a polysaccharide) provided a remarkable protective and therapeutic effect in skin disorders caused by a skin barrier dysfunction..

The histological examination also revealed that the skin from control mice treated with the topical compositions of the invention did not differ from sham controls.

### B) Immunostaining for tight junctions (TJ) and filaggrin

Tight junctions, also known as occluding junctions or zonulae occludentes (singular, zonula occludens), are the closely associated areas of two cells whose membranes join together forming a virtually impermeable barrier to fluid.

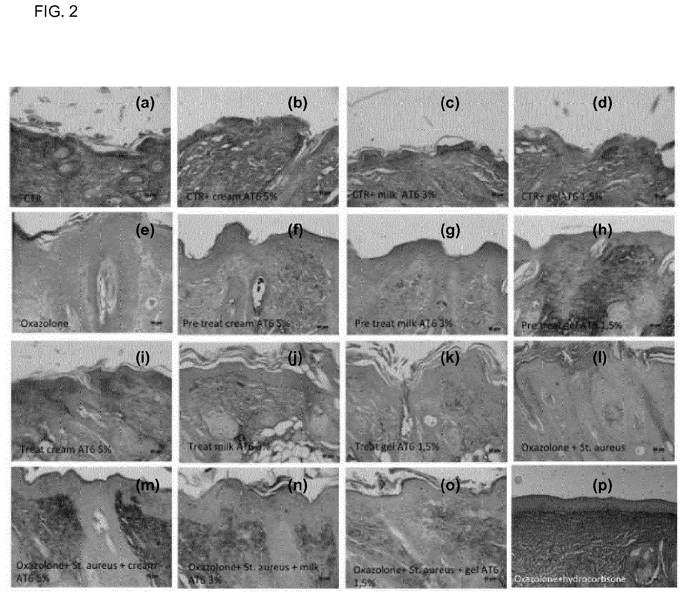

As shown in FIG. 2 (ZO-1) and 3 (occludin), oxazolone treatment induced an increase of TJ permeability throughout the entire skin. On the contrary, a significant reduction of the alteration of zonula occludens (ZO)-1 and occludin localization by immunohistochemistry was observed in AT-6 treated mice.

The immunohistological analysis for St. aureus-induced dermatitis showed a remarkably reduction of TJ staining after bacterial infection.

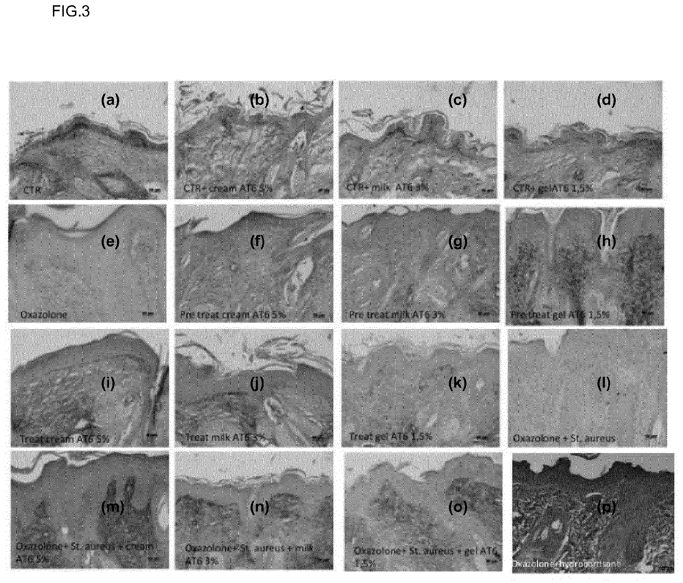

All the formulations were able to recover ZO-1 and occludin positive staining. (Figure 2 and 3 respectively). That is, the topical administration of AT-6 appropriately restored tight junctions.

On the other hand, filaggrin (filament aggregating protein) is a filament-associated protein that binds to keratin fibers in epithelial cells. Within the stratum corneum, filaggrin monomers can become incorporated into the lipid envelope, which is responsible for the skin barrier function.

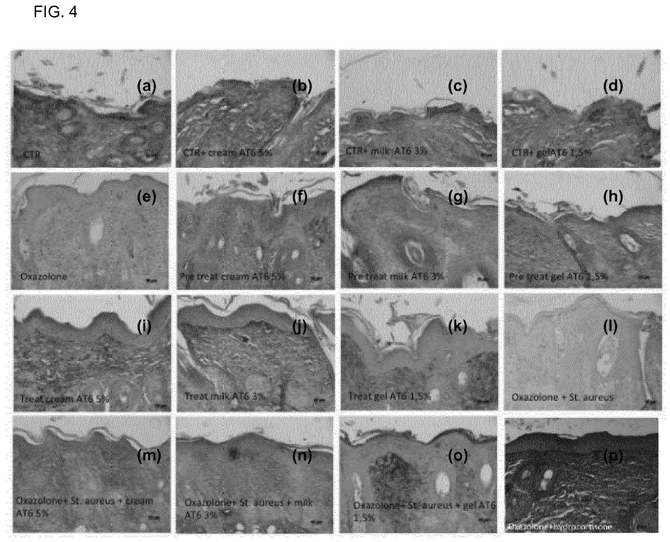

As it is shown in FIG. 4, the topical administration of AT-6 is able to restore filaggrin positive staining compared to oxazolone.

Therefore, the data provided are indicative that the topical administration of AT-6 allows restoring the skin barrier in a model of atopic dermatitis induced by oxazolone.

### C) Eryhtema index

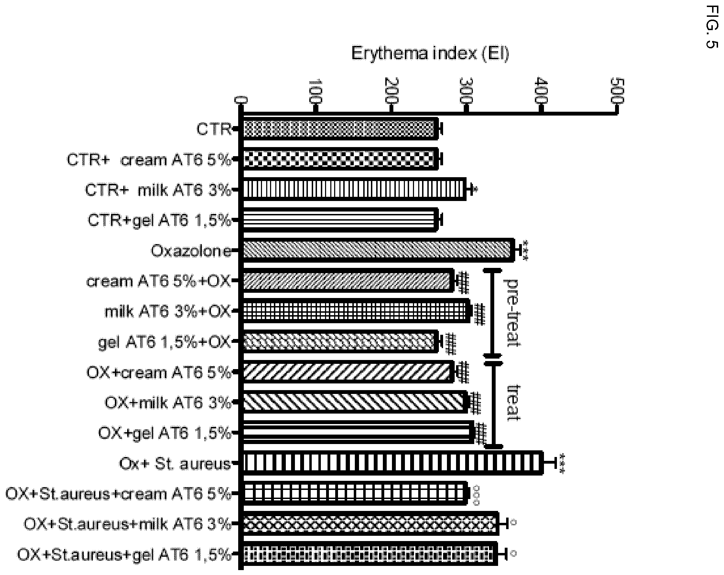

Oxazolone prominently increased erythema index while mice that received AT-6 by topic administration revealed a markedly reduced erythema in skin lesions (FIG. 5).

### D) Mast cells quantification

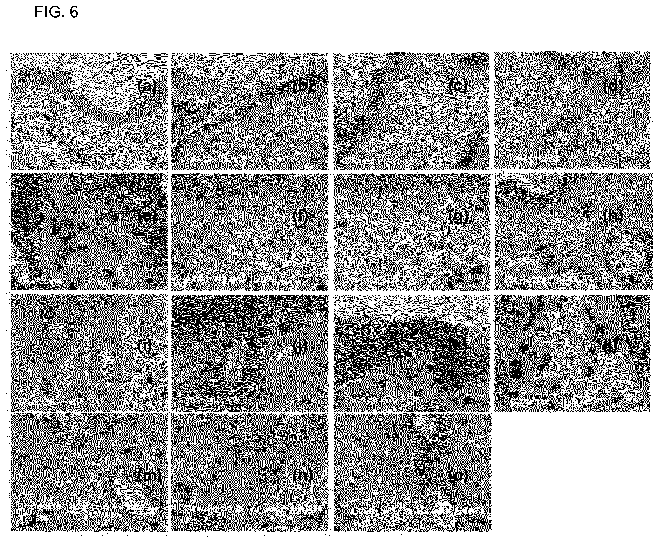

Treatment with oxazolone and *S. aureus* infection caused an increase in mast cell degranulation as determined by toluidine blue staining in the skin lesion (FIG. 6). The pre-treatment and treatment with a combination of pea protein and a polysaccharide showed a markedly decreasing of mast cells number.

The control mice treated with all formulations were comparable to sham groups (FIG. 6).

The staining for St. aureus-induced dermatitis showed a notably increasing of mast cells after St. aureus infection. All formulations were able to reduce mast cell degranulation, even if the AT6 cream 5% showed the highest significance (FIG. 6).

### E) ELISA results

The Labelling Index(LI) of BrdU-labelled cell in the lymph node was calculated from the number of stained BrdU-positive cells per 100 cells counted in the cortex, paracortex and medulla. In each lymph node, cells were counted at each standardized site, at three sites of cortex, paracortex and medulla, without prior knowledge of the treatment. The LI of the BrdU-labeled cells in the epidermis was calculated from the number of stained BrdU-positive cells per 100 epidermal cells counted from the epidermis for each section

| Samples | SI (Stimulation Index) |
|---|---|
| CTR+ cream AT6 5% | 1 |
| CTR +lotion AT6 3% | 1,1 |
| CTR+ gel AT6 1,5% | 1 |
| PBS | 1 |

The stimulation index (SI) was calculated from the fold increase of the total number of positive BrdU LNCs in a lymph node compared to that of the respective vehicle control groups.

A substance was considered positive (irritant) if the value of SI was greater than or equal to 1.6.

Therefore, all the formulations tested can be considered as not sensitizing.

### 4. Effect of a topical combination comprising xyloglucan and pea protein in the treatment of psoriasis

### 4.1 Animals

Six- to eight-week-old, male BALB/c mice (ENVIGO, Milan, Italy) were housed in a controlled environment (22 ± 2 °C, 55 ± 15% relative humidity, 12 h light/dark cycle). After a one- week acclimation, mice were fed a standard diet and water. The animals used for this study were randomly selected from those suitable, available at that time.

The mice were divided into five experimental groups:
Group 1: the mice received Vaseline cream without Imiquimod (IMQ) treatment for 1 week (n= 5);
Group 2: the mice received AT-6 cream without imiquimod (IMQ) treatment for 1 week (n = 10);
Group 3: the mice received vehicle with imiquimod treatment 1 week (n = 10);
Group 4: mice received AT-6 cream 1h before Imiquimod (IMQ) treatment for 1 week (n= 10); and
Group 5: mice received AT-6 cream with 4h after Imiquimod (IMQ) treatment for 1 week (n= 10); and
AT-6 cream is the one provided under section 2.1. above.

### 4.2. Animal model damage

All measurements and topical applications were conducted under isoflurane anesthesia, while the mice lay on a heating mat. To facilitate dermal application and measurements, the right flank were clipped on an area of 2.0 x 2.0 cm and chemically depilated two days prior to the start of experiments with hair removal cream for 4 min.

Psoriasis-like dermatitis were induced on the right ear and on a 1.5 × 1.5 cm area of the depilated right flank by Imiquimod (IMQ) (62.5 mg, Aldara® 5% cream, Meda AB, Solna, Sweden) application for seven days.

The combination of the invention was topically applied daily for five days (from days 0 to 4) on the right flank (44.5 µL) and right ear (19.8 µL) using a nylon mesh for even distribution of aqueous solutions.

AT-6 cream was topically applied 1h before imiquimod treatment. Following a 3-min massage, the test substance was allowed to dry and seep in for 1 h. Sham psoriasis animals were challenged with vaseline cream.

### 4.3. Results

### 4.3.1. Histological evaluation:

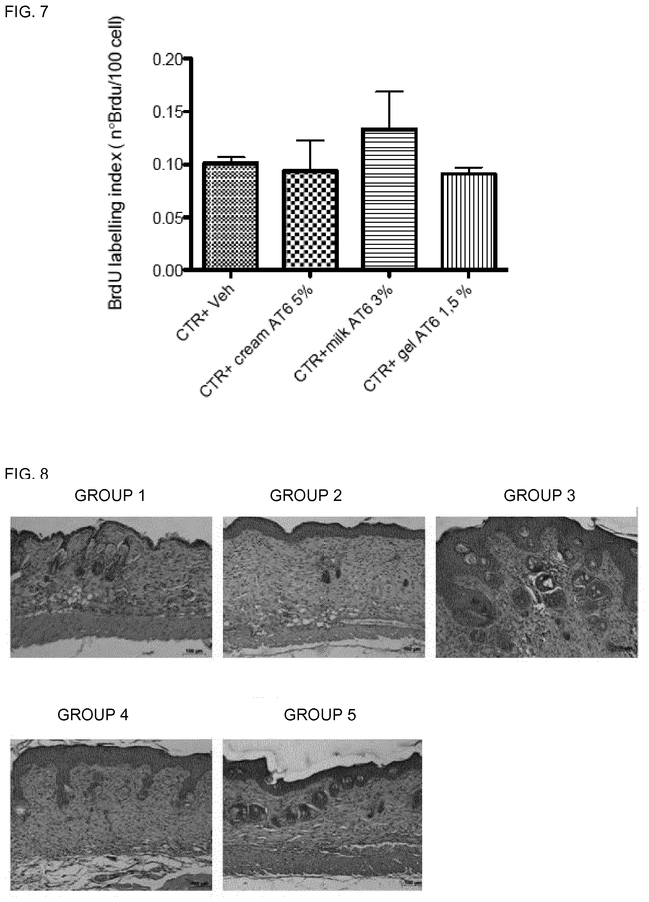

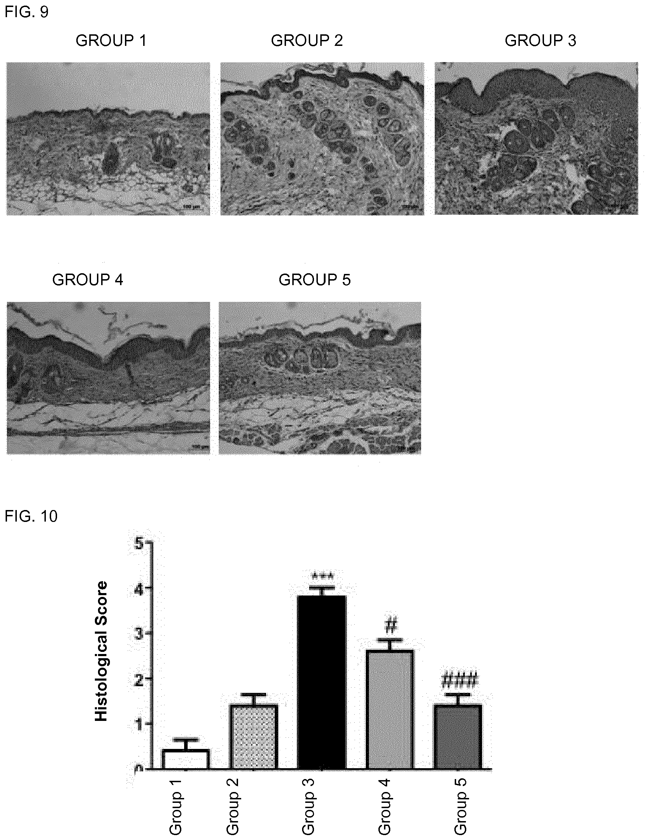

Histological examination of right flank, following the same protocol as the one provided under section 3.1. above, revealed characteristic pathological changes psoriasis-like after imiquimod treatment. Pre-treatment with AT-6 cream (Group 4) well reduced the degree of tissue injury, whereas the post-treatment significantly reduced the pathological sign of psoriasis in skin sections from both the right flank and ear (FIG. 8 and 9, respectively).

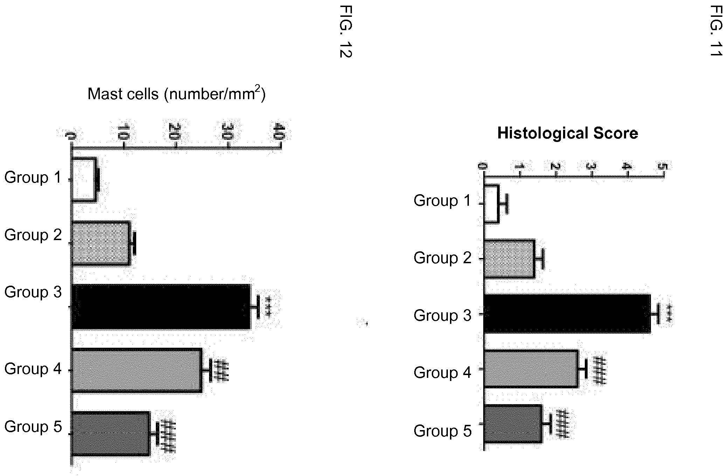

The histological scores for both the right flank and ear (FIG. 10 and 11, respectively) were made by an independent observer as explained in previous sections.

### 4.3.2. Mast cell quantification:

Following the protocol provided under section 3.1, it was found that the treatment with imiquimod caused an increase in mast cell degranulation as determined by toluidine blue staining in the lesioned right flank and ear. AT-6 cream in both pre- and post-treatment markedly reduced the degranulation of mast cells in both tissue
(FIG.s 12 and 13, respectively).

### 4.3.3. Erythema index and ear thickness

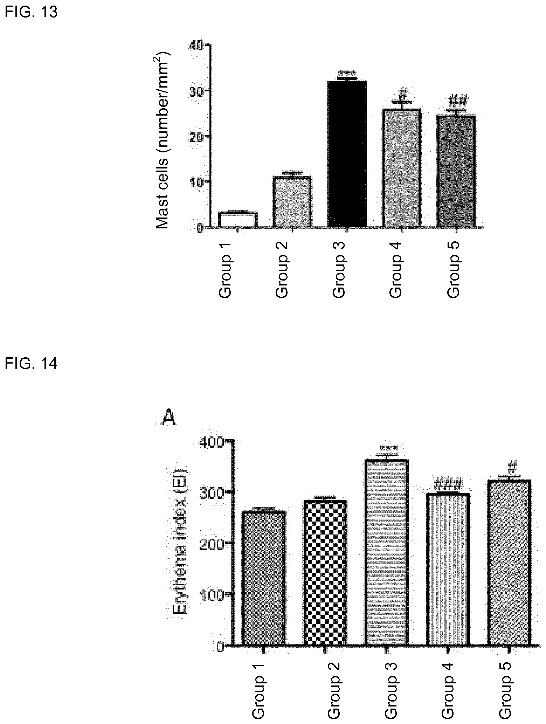

Following the protocol provided under section 3.1, it was found that imiquimod treatment notably increased erythema index (calculated as explained above) and the thickeness of skin in psoriasis-like mouse model compared to sham group, while mice that received AT-6 cream, in both pre- and post-treatment, revealed a markedly reduced erythema index in the lesioned flank (FIG. 14).

Ear thickness was markedly increased in the psoriasis-like mouse model induced by imiquimod compared to healthy skin. However the topical application of AT-6 cream significantly reduced psoriasic phenotype in both pre- and post-treatment.

### 4.4. Conclusion:

This study clearly has shown that a combination of pea protein and a polysaccharide, such as xyloglucan, was able to prevent and ameliorate significantly the clinical signs of psoriasis, characterized by erythema, scaling and epidermal thickening.

### 5. Effect of a topical combination comprising xyloglucan and pea protein in the treatment of rosacea

### 5.1. Animals

Twenty four-week-old female BALB/c mice were purchased from Envigo (Milan, Italy) and acclimatized for 1 week before initiating the experiments. They were housed in a controlled environment (22 ± 2 °C, 55 ± 15% relative humidity, 12 h light/dark cycle). After a one-week acclimation, mice were fed a standard diet and water. The animals used for this study were randomly selected from those suitable, available at that time.
60 mice were divided into six experimental groups:
Group 1 received vehicle (PBS) without LL-37 treatment for 2 days (n = 4);
Group 2 received AT-6 cream without LL-37 treatment for 2 days (n = 8);
Group 3 received vehicle (PBS) with LL-37 treatment for 2 days (n = 8);
Group 4 received AT-6 cream immediately after LL-37 treatment, twice for 2 days (n= 8); and
Group 5 received AT-6 cream 1h before LL-37 treatment, twice for 2 days (n= 8).
AT-6 cream is the one provided under section 2.1. above.

### 5.2. Animal model damage

Rosacea-like skin lesion was induced using the LL-37 peptide (SEQ ID NO 1: LLGDFFRKSKEKIGKEFKRIVQRIKDFLRNLVPRTES) as described previously (Yamasaki et al., 2007). The hair on the backs of the BALB/c mice were shaved using an electric shaver. Twenty-four hours later, 40 µl of LL-37 in nanopure water (320 µm; InvivoGen, San Diego, CA, USA) was injected intradermally (i.d.) into the shaved area using a 0.5 mL insulin syringe (31 gauge), resulting in the formation of a dermal bleb. Control mice were injected i.d. with 40 µL of nanopure water alone.

### 5.3. Results

### 5.3.1. Redness score:

Forty-eight hours after the initial injection, the dorsal skin were photographed. The severity of rosacea-like skin lesions were evaluated based on the redness score and area. The redness were scored from 1 to 5, with 5 being the reddest. The redness area was assessed by stereomicroscope measurements (Leica M50; Leica, Wetzlar, Germany).

LL-37 treatment prominently increased redness in the dorsal skin while mice that received AT-6 cream in pre- and post- treatment, revealed a marked reduction of redness in skin lesioned area.

### 5.3.2. Histological analysis:

Histological examination of skin, as explained above under item 3.1. above, revealed characteristic pathological changes after LL-37 treatment compared to sham groups. The pre- and post- treatment with AT-6 cream significantly reduced the major skin characteristic of rosacea.

### 5.3.3. Immunohistochemical analysis of CD4+ and CD8+:

Immunostaining evaluation of CD4+ and CD8+ T cell response ((Santa Cruz Biotechnology, sc-514571, and sc-7970 respectively) to rosacea revealed that both CD4+and CD8+ positive staining were significantly increased in mice following LL-37 treatment compared to sham groups. The pre-treatment and post-treatment with AT-6 cream significantly decreased CD4+ and CD8+ positive staining.

### 5.3.4. Immunohistochemical analysis of IL-18

IL-18 plays an important role in cutaneous inflammation included rosacea. Immunohistochemistry analysis showed a significant increased of IL-18 positive staining (Santa Cruz Biotechnology, sc-7954) in LL-37 treated mice compared to control group. Ding et al. criteria (Ding et al., 2012) was used, which included assignment of the intensity of staining using a scale of 0-10 (with 0 indicating a lack of brown immunoreactivity and 10 reflecting intense dark brown staining) by three observers.

Immunohistochemistry analysis showed a significant increased of IL-18 positive staining in LL-37 treated mice compared to control group. On the contrary, AT-6 cream in post- and pre-treatments reduced significantly IL-18 positive staining.

### 5.4. Conclusions

This study clearly has shown that the combination of the invention was able to ameliorate the clinical signs of rosacea in terms of morphological and biochemical modifications in both pre- and post-treatments.

### 6. Comparative test

In order to confirm whether the surprising improved properties reported in the above examples were due to the combination of pea protein and a polysaccharide, the following test was performed to compare the behaviour of pea protein ("PP"), xyloglucan ("XYL") and of the combination AT-6 (PP+XYL) in an atopic dermatitis model.

### 6.1. Animals

Specific pathogen-free 5-week-old female SKH-1 hairless mice (Harlan, Milan, Italy) were housed in a controlled environment (22 ± 2 °C, 55 ± 15% relative humidity, 12 h light/dark cycle). After one-week acclimation, mice were fed a standard diet and water. Animal experiments is in compliance with Italian regulations on protection of animals used for experimental and other scientific purposes (DM 116192) as well as EU regulations (OJ of EC L 358/1 12/18/1986). The animals used for this study were randomly selected from those suitable, available at that time.

55 mice were divided into 6 experimental groups:
Group 1 mice received vehicle (polysorbate 80) without oxazolone treatment for 4 weeks (n = 5);
Group 2 mice received pea protein (0.14 g/mouse by topical administration) without oxazolone treatment for 4 weeks (n = 10);
Group 3 mice received xyloglucan (0.06 g/mouse by topical administration) without oxazolone treatment for 4 weeks (n = 10);
Group 4 mice received vehicle (polysorbate 80) with oxazolone treatment for 4 weeks (n = 10);
Group 5 mice received pea protein 1h before oxazolone treatment for 4 weeks (n= 10); and
Group 6 mice received xyloglucan 1h before oxazolone treatment for 4 weeks (n= 10).

The pea protein formulation ("PP") was prepared by dissolving the Pea protein powder in polysorbate 80.

The xyloglucan formulation ("XYL") was prepared by dissolving the xyloglucan powder in polysorbate 80.

### 6.2. Animal model damage

Mice were topically treated with 200 µl of 0.5% oxazolone (Sigma- Aldrich, St. Louis, MO, USA) to the dorsal skin three times a week for four weeks. Induction of AD were confirmed through macroscopic observation (e.g., eruption and erythema).

### 6.3. Results

The protocols used for the histological evaluation, mast cell granulation and filaggrin expression are the same as those referred under section 3.1. above.

### 6.3.1 Histological evaluation:

Histological examination of skin revealed characteristic pathological changes after oxazolone treatment compared to sham group.

The pre-treatment with PP and XYL, 1h before oxazolone administration, showed a maintenance of tissue architecture.

Both PP and XYL were able to reduce the skin lesion. The histological score was made by an independent observer, as explained above. For each one the groups the mean value was determined.

### 6.3.2. Immunostaining for filaggrin:

The control mice treated with PP and XYL were comparable to sham groups. Oxazolone treatment reduced the positive staining for filaggrin. PP and XYL were able to restore filaggrin positive staining compared to oxazolone. For each one the groups the mean value was determined.

### - Mast cells quantification:

Treatment with oxazolone caused an increase in mast cell degranulation as determined by toluidine blue staining in the skin lesion. The pre-treatment with PP and XYL showed a markedly decrease of mast cells number. The pre-treatment with PP and XYL, 1h before oxazolone administration, showed a significant decreasing of mast cells degranulation by topical administration. The control mice treated with both compounds were comparable to sham groups. For each one the groups the mean value was determined.

The obtained results were compared with those provided in Example 1 above (for pre-treatment), wherein mice received 0.14g PP+0.06g XYL. Table 1 summarizes the results:

**Table 1**

| | oxazolone | oxazolone+PP (0.14 g/mouse) | oxazolone+XYL (0.06 g/mouse) | oxazolone+AT-6 (0.2 g of PP+XYL/mouse) |
|---|---|---|---|---|
| Histological score | 4.7±0.48 | 1.68±0.36 | 3,9±0.20 | **1.5±0.56** |
| Mast cells | 45.9±3.4 | 34.00±1.96 | 41.6±0.93 | **20.88±4.48** |
| Filaggrin | 1.7±0.48 | 2.94±0.55 | 2.6±0.19 | **5±0.75** |

From the above table it can be concluded that a remarkable improved effect was found when pea protein was topically administered in combination with the polysaccharide: (a) the histological score was reduced, (b) the expression of filaggrin was substantially increased; and (c) the mast cell infiltration was substantially reduced. In fact, the effect achieved with the combination of the invention was higher than the effect achieved by each one of the components (pea protein or xyloglucan) separately, especially with respect to the number of mast cells, which supports the surprising reducing mast cell degranulation effect of the combination of the invention.

### Citation List

### Non Patent Literature

Audrey R. et al., "Structure and Functional Properties of Ulvan, a Polysaccharide from Green Seaweeds", 2007, American Chemical Society, 8(6), 1765-1774;
Ding W. et al., "Cell-specific expression and immunolocalization of nitric oxide synthase isoforms and soluble guanylyl cyclase α1 and β1 subunits in the ovary of fetal, neonatal and immature pigs", Anim. Reprod. Sci., 2012, v. 131, pages172-180;
Dorota Purzycka-Bohdan et al., "Genetic background of skin barrier dysfunction in the pathogenesis of psoriasis vulgaris", Postepy Dermatol Alergol., 2015, 32(2), 123-126;
Flavia Alvim Sant'Anna Addor "Skin barrier in rosacea", An Bras Dermatol., 2016, vol. 91(1), 59-63;
Grainne M. O. et al., "Filaggrin in atopic dermatitis", J. Allergy Clin. Immunol., 2008, vol.122(4), 689-693;
Baoru Yin et al., "Influence of pea protein aggregates on the structure and stability of pea protein/soybean polysaccharide complex emulsions", 2015, Molecules, 20(3), 5165-5183; Kim M-S. et al., "Panduratin A, an activator of PPAR-α/δ, suppresses the development of oxazolone-induced atopic dermatitis-like symptoms in hairless mice", 2014, Life Sciences, 100(1):45-54;
Nolte T. et al., "Induction of oxazolone-mediated features of atopic dermatitis in NODscid IL2Rγ mice engrafted with human peripheral blood mononuclear cells", 2013, Disease Models and Mechanisms, 6(1): 125-134; and
Yamasaki K. et al., "Increased serine protease activity and cathelicidin promotes skin inflammation in rosacea", 2007, Nat. Med., 13(8): 975-980

## Claims

1. A combination comprising pea protein and a polysaccharide for use in the topical treatment or prevention of a skin disorder due to a skin barrier dysfunction.

2. The combination for use according to claim 1, wherein the skin disorder is an inflammatory skin disorder.

3. The combination for use according to any one of the claims 1-2, wherein the skin barrier dysfunction is selected from atopic dermatitis, psoriasis and rosacea.

4. A topical pharmaceutical composition for use in the topical treatment or prevention of a skin disorder due to a skin barrier dysfunction, the composition comprising:
(i) an effective amount of a combination comprising pea protein and polysaccharide; and
(ii) one or more appropriate topical pharmaceutically acceptable excipients or carriers.

5. The topical pharmaceutical composition for use according to claim 4 which is a gel, lotion or cream.

6. The topical pharmaceutical composition for use according to any one of the claims 4-5, which is in the form of a patch.

7. The combination for use according to any one of the claims 1-3 or the topical composition for use according to any of the claims 4-6, wherein the combination comprising pea protein and polysaccharide is selected from a mixture of pea protein and a polysaccharide; and a pea protein-polysaccharide conjugated product.

8. The combination for use or the topical composition for use according to any of the preceding claims, wherein the combination comprising pea protein and polysaccharide is a pea protein-polysaccharide conjugated product.

9. The combination for use or the topical composition for use according to any of the preceding claims, wherein the combination is a pea-protein polysaccharide conjugated product obtainable by a process comprising:
(a) preparing a mixture comprising pea protein, a polysaccharide, and an appropriate polar solvent; wherein:
- the weight ratio between polysaccharide and pea protein is comprised from 20:80 to 60:40, and
- the pH of the solution is comprised from 8.0 to 10.5; and
(b) performing a Maillard reaction by heating the solution resulting from step (a) at an appropriate temperature for the necessary period of time to conjugate the protein and the polysaccharide.

10. The combination for use or the topical composition for use according to any of the preceding claims, wherein the polysaccharide is xyloglucan, fucoidan or ulvan.

11. The combination for use or the topical composition for use according to any of the preceding claims, wherein the weight ratio polysaccharide:pea protein:pea protein is comprised in the range from 25:75 to 55:45.

12. The combination for use or the topical composition for use according to any of the preceding claims, wherein the weight ratio of polysaccharide:pea protein is 30:70.

13. The combination for use or the topical composition for use according to any of the preceding claims, wherein the combination comprising pea protein and a polysaccharide is a conjugated product, the conjugated product being obtainable by a process comprising the steps of:
(i) mixing the pea protein with water;
(ii) adjusting the pH of the solution resulting from step (i) to a value of 9.5 to 10.5;
(iii) adding the xyloglucan to the solution resulting from step (ii); and
(iv) performing Maillard reaction by heating the solution resulting from step (iii) at a temperature comprised from 30 to 190 °C for the necessary period of time to conjugate the protein and the xyloglucan; and
being the weight ratio of xyloglucan:pea protein of 30:70.

14. The combination for use or the topical composition for use according to any of the preceding claims, wherein wherein the combination comprising pea protein and a polysaccharide is a conjugated product, the conjugated product being obtainable by a process comprising the steps of:
(a) preparing a mixture comprising pea protein, polysaccharide, and water, the weight ratio between polysaccharide and pea protein being 30:70, and the pH of the solution being comprised from 9.5 to 10.5, and
(b) performing Maillard reaction by heating the solution resulting from step (a) at a temperature of 160 °C.

15. A topical medical device comprising the topical pharmaceutical composition for use as defined in any one of the claims 4-14.

## Patentansprüche

1. Eine Kombination umfassend Erbsenprotein und ein Polysaccharid zur Verwendung bei der topischen Behandlung oder Prävention von einer durch eine Störung der Hautbarriere verursachten Hauterkrankung.

2. Die Kombination zur Verwendung nach Anspruch 1, wobei die Hauterkrankung eine entzündliche Hauterkrankung ist.

3. Die Kombination zur Verwendung nach einem der Ansprüche 1-2, wobei die Störung der Hautbarriere aus atopischer Dermatitis, Psoriasis und Rosazea ausgewählt ist.

4. Eine topische pharmazeutische Zusammensetzung zur Verwendung bei der topischen Behandlung oder Prävention von einer durch eine Störung der Hautbarriere verursachten Hauterkrankung, wobei die Zusammensetzung folgendes umfasst:
(i) eine wirksame Menge von einer Kombination umfassend Erbsenprotein und Polysaccharid; und
(ii) eine(n) oder mehrere geeignete topische pharmazeutisch akzeptable Hilfsstoffe oder Trägersubstanzen.

5. Die topische pharmazeutische Zusammensetzung zur Verwendung nach Anspruch 4, welche ein Gel, eine Lotion oder eine Creme ist.

6. Die topische pharmazeutische Zusammensetzung zur Verwendung nach einem der Ansprüche 4-5, welche in Form eines Pflasters ist.

7. Die Kombination zur Verwendung nach einem der Ansprüche 1-3 oder die topische Zusammensetzung zur Verwendung nach einem der Ansprüche 4-6, wobei die Kombination umfassend Erbsenprotein und Polysaccharid aus einer Mischung aus Erbsenprotein und einem Polysaccharid und einem konjugierten Produkt aus Erbsenprotein-Polysaccharid ausgewählt ist.

8. Die Kombination zur Verwendung oder die topische Zusammensetzung zur Verwendung nach einem der vorangehenden Ansprüche, wobei die Kombination umfassend Erbsenprotein und Polysaccharid ein konjugiertes Produkt aus Erbsenprotein-Polysaccharid ist.

9. Die Kombination zur Verwendung oder die topische Zusammensetzung zur Verwendung nach einem der vorangehenden Ansprüche, wobei die Kombination ein konjugiertes Produkt aus Erbsenprotein-Polysaccharid ist, welches durch ein Verfahren erhalten werden kann, das folgendes umfasst:
(a) herstellen von einer Mischung umfassend Erbsenprotein, ein Polysaccharid und ein geeignetes polares Lösungsmittel; wobei:
- das Gewichtsverhältnis vom Polysaccharid zum Erbsenprotein von 20:80 bis 60:40 reicht, und
- der pH-Wert der Lösung von 8,0 bis 10,5 reicht; und
(b) durchführen von einer Maillard-Reaktion durch Erhitzung der aus dem Schritt (a) erhaltenen Lösung bei einer geeigneten Temperatur während des nötigen Zeitraums zur Konjugation des Proteins und des Polysaccharids.

10. Die Kombination zur Verwendung oder die topische Zusammensetzung zur Verwendung nach einem der vorangehenden Ansprüche, wobei das Polysaccharid Xyloglucan, Fucoidan oder Ulvan ist.

11. Die Kombination zur Verwendung oder die topische Zusammensetzung zur Verwendung nach einem der vorangehenden Ansprüche, wobei das Gewichtsverhältnis Polysaccharid: Erbsenprotein im Bereich von 25:75 bis 55:45 liegt.

12. Die Kombination zur Verwendung oder die topische Zusammensetzung zur Verwendung nach einem der vorangehenden Ansprüche, wobei das Gewichtsverhältnis Polysaccharid:Erbsenprotein 30:70 beträgt.

13. Die Kombination zur Verwendung oder die topische Zusammensetzung zur Verwendung nach einem der vorangehenden Ansprüche, wobei die Kombination umfassend Erbsenprotein und ein Polysaccharid ein konjugiertes Produkt ist, wobei das konjugierte Produkt durch ein Verfahren erhalten werden kann, das folgende Schritte umfasst:
(i) mischen des Erbsenproteins mit Wasser;
(ii) einstellen des pH-Werts der aus dem Schritt (i) erhaltenen Lösung auf einen Wert von 9,5 bis 10,5;
(iii) hinzufügen des Xyloglucans der aus dem Schritt (ii) erhaltenen Lösung; und
(iv) durchführen der Maillard-Reaktion durch Erhitzung der aus dem Schritt
(iii) erhaltenen Lösung bei einer Temperatur, die von 30 bis 190 °C reicht, während des nötigen Zeitraums zur Konjugation des Proteins und des Xyloglucans; und
wobei das Gewichtsverhältnis Xyloglucan:Erbsenprotein 30:70 beträgt.

14. Die Kombination zur Verwendung oder die topische Zusammensetzung zur Verwendung nach einem der vorangehenden Ansprüche, wobei die Kombination umfassend Erbsenprotein und ein Polysaccharid ein konjugiertes Produkt ist, wobei das konjugierte Produkt durch ein Verfahren erhalten werden kann, das folgende Schritte umfasst:
(a) herstellen von einer Mischung umfassend Erbsenprotein, Polysaccharid und Wasser, wobei das Gewichtsverhältnis vom Polysaccharid zum Erbsenprotein 30:70 beträgt und der pH-Wert der Lösung von 9,5 bis 10,5 reicht, und
(b) durchführen der Maillard-Reaktion durch Erhitzung der aus dem Schritt (a) erhaltenen Lösung bei einer Temperatur von 160 °C.

15. Eine topische medizinische Vorrichtung umfassend die topische pharmazeutische Zusammensetzung zur Verwendung wie in einem der Ansprüche 4-14 definiert.

## Revendications

1. Une combinaison comprenant de la protéine de pois et un polysaccharide pour l'utilisation dans la prévention ou le traitement topique d'un trouble de la peau dû à un dysfonctionnement de la barrière cutanée.

2. La combinaison pour l'utilisation selon la revendication 1, dans laquelle le trouble de la peau est un trouble inflammatoire de la peau.

3. La combinaison pour l'utilisation selon l'une quelconque des revendications 1-2, dans laquelle le dysfonctionnement de la barrière cutanée est choisi parmi la dermatite atopique, le psoriasis et la rosacée.

4. Une composition pharmaceutique topique pour l'utilisation dans la prévention ou le traitement topique d'un trouble de la peau dû à un dysfonctionnement de la barrière cutanée, la composition comprenant :
(i) une quantité efficace d'une combinaison comprenant de la protéine de pois et du polysaccharide ; et
(ii) un ou plusieurs excipients ou véhicules topiques pharmaceutiquement acceptables.

5. La composition pharmaceutique topique pour l'utilisation selon la revendication 4 qui est un gel, une lotion ou une crème.

6. La composition pharmaceutique topique pour l'utilisation selon l'une quelconque des revendications 4-5, qui est en forme d'un timbre.

7. La combinaison pour l'utilisation selon l'une quelconque des revendications 1-3 ou la composition topique pour l'utilisation selon l'une quelconque des revendications 4-6, dans laquelle la combinaison comprenant de la protéine de pois et du polysaccharide est choisie d'un mélange de protéine de pois et un polysaccharide ; et un produit conjugué de protéine de pois-polysaccharide.

8. La combinaison pour l'utilisation ou la composition topique pour l'utilisation selon l'une quelconque des revendications précédentes, dans laquelle la combinaison comprenant de la protéine de pois et du polysaccharide est un produit conjugué de protéine de pois-polysaccharide.

9. La combinaison pour l'utilisation ou la composition topique pour l'utilisation selon l'une quelconque des revendications précédentes, dans laquelle la combinaison est un produit conjugué de protéine de pois-polysaccharide pouvant être obtenu moyennant un procédé comprenant :
(a) préparer un mélange comprenant de la protéine de pois, un polysaccharide, et un solvant polaire approprié ; où :
- le rapport en poids du polysaccharide à la protéine de pois est compris de 20 : 80 à 60 : 40, et
- le pH de la solution est compris de 8,0 à 10,5 ; et
(b) effectuer une réaction de Maillard en échauffant la solution résultant de l'étape (a) à une température appropriée pendant la période nécessaire pour conjuguer la protéine et le polysaccharide.

10. La combinaison pour l'utilisation ou la composition topique pour l'utilisation selon l'une quelconque des revendications précédentes, dans laquelle le polysaccharide est du xyloglucane, du fucoïdane ou de l'ulvane.

11. La combinaison pour l'utilisation ou la composition topique pour l'utilisation selon l'une quelconque des revendications précédentes, dans laquelle le rapport en poids polysaccharide : protéine de pois est compris dans l'intervalle allant de 25 : 75 à 55 : 45.

12. La combinaison pour l'utilisation ou la composition topique pour l'utilisation selon l'une quelconque des revendications précédentes, dans laquelle le rapport en poids de polysaccharide : protéine de pois est 30 : 70.

13. La combinaison pour l'utilisation ou la composition topique pour l'utilisation selon l'une quelconque des revendications précédentes, dans laquelle la combinaison comprenant de la protéine de pois et un polysaccharide est un produit conjugué, le produit conjugué pouvant être obtenu moyennant un procédé comprenant les étapes de :
(i) mélanger la protéine de pois avec de l'eau ;
(ii) ajuster le pH de la solution résultant de l'étape (i) à une valeur de 9,5 à 10,5;
(iii) ajouter le xyloglucane à la solution résultant de l'étape (ii); et
(iv) effectuer la réaction de Maillard en échauffant la solution résultant de l'étape (iii) à une température comprise de 30 à 190 °C pendant la période nécessaire pour conjuguer la protéine et le xyloglucane ; et
étant le rapport en poids de xyloglucane : protéine de pois de 30 : 70.

14. La combinaison pour l'utilisation ou la composition topique pour l'utilisation selon l'une quelconque des revendications précédentes, dans laquelle la combinaison comprenant de la protéine de pois et un polysaccharide est un produit conjugué, le produit conjugué pouvant être obtenu moyennant un procédé comprenant les étapes de :
(a) préparer un mélange comprenant de la protéine de pois, du polysaccharide, et de l'eau, le rapport en poids du polysaccharide à la protéine de pois étant 30 : 70, et le pH de la solution étant compris de 9,5 à 10,5, et
(b) effectuer la réaction de Maillard en échauffant la solution résultant de l'étape (a) à une température de 160 °C.

15. Un dispositif médical topique comprenant la composition pharmaceutique topique pour l'utilisation telle que définie dans l'une quelconque des revendications 4-14.
